# DEMANDE DE BREVET EUROPEEN

(11) **EP 0 591 030 A2**
(43) Date de publication de la demande: **06.04.1994**
(21) Numéro de dépôt: 93402323.5
(22) Date de dépôt: 23.09.1993
(51) Int. Cl.: C07D 401/04, C07D 403/04, A61K 31/395

(54) **1-Hétéroaryl-azétidines et -pyrrolidines comme 5-HT3 agonistes**

(30) Priorité: 25.09.1992 EP 92402642; 25.09.1992 EP 92402643
(71) Demandeur: SANOFI, F-75008 Paris (FR); MIDY S.p.A., I-20137 Milano (IT)
(72) Inventeur: Guzzi, Umberto, I-20100 Milan (IT); Giudice, Antonina, I-20154 Milan (IT); Mazza, Vivian, Legnano (Milan) (IT); Baroni, Marco, I-20010 Vanzago (Milan) (IT); Landi, Marco, Bussero (Milan) (IT)
(74) Mandataire: Le Roux, Martine

(57) **Abrégé**

La présente invention concerne de nouvelles 1-hétéroaryl-azétidines et -pyrrolidines, douées d'activité 5-HT₃ agoniste, de formule (I) 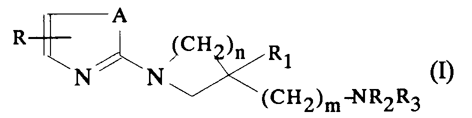
dans laquelle
- A: représente un groupement -CH=CH-, -CH=N- ou -N=CH-;
- R: représente un atome d'hydrogène, un atome d'halogène, un groupe (C₁-C₄)alkyle, (C₁-C₄)alcoxy, (C₁-C₄)alkylthio, un groupe cyano, carboxamido, trifluorométhyle, vinyle, formyle, un groupe carboxy libre, salifié ou estérifié, un groupe hydroxy, hydroxyméthyle, mercapto, un groupe amino, mono- ou di-(C₁-C₄)alkylamino, aminométhyle, mono- ou di-(C₁-C₄)alkylamino- méthyle, 1-pipéridino, 1-pyrrolidino, 1-pipérazino ou 4-(C₁-C₄)alkyl-1-pipérazino, ce groupe R pouvant substituer un quelconque des atomes d'hydrogène du noyau hétéroarylique;
- R₁: est un atome d'hydrogène ou un groupe méthyle;
- R₂ et R₃,: identiques ou différents, représentent un atome d'hydrogène ou un groupe (C₁-C₄)alkyle;

n est 1 ou 2, m est 1 ou 0, et m +n ≧ 2;
ainsi que leurs sels d'addition avec des acides minéraux ou organiques.
L'invention concerne aussi le procédé de préparation de ces nouveaux composés, les intermédiaires du procédé et l'utilisation des composés (I) à titre de médicaments.

## Description

La présente invention concerne de nouveaux dérivés 1-hétéroaryl-azétidiniques et -pyrrolidiniques doués d'activité agoniste vis-à-vis des récepteurs 5-HT₃.

L'invention concerne aussi le procédé de préparation de ces composés, les nouveaux intermédiaires obtenus dans ce procédé, l'application desdits nouveaux composés à titre de médicaments et les compositions pharmaceutiques les renfermant.

Des dérivés pyraziniques, pyrimidiniques et pyridaziniques qui peuvent être substitués inter alia par un radical 2- ou 3-azétidinyle ont été décrits dans la demande de brevet européen EP-A-327155 comme stimulants des récepteurs muscariniques centraux.

Des quinolines et des naphtyridines substituées par un radical 1-azétidinyle, utiles comme bactéricides, ont été décrites dans les demandes de brevet européen EP-A-106489 et EP-A-153163.

Des dérivés azétidiniques substitués sur l'atome d'azote par un hétérocycle et en position 3- par un groupe amino substitué ont été revendiqués dans la demande de brevet allemand DE-A-2241097 où ils sont décrits comme analgésiques et antiinflammatoires.

Le brevet européen EP-155870 cite l'utilisation de la 3-aminoazétidine, qui est revendiquée, dans la préparation de la 3-amino-1-(6-chloropyrid-2-yl)azétidine douée d'activité anorexigène.

Des dérivés de l'acide 3-pyridinecarboxylique, substitués à la position 2- par un groupe fluorophénylamino, à la position 5- par un atome de fluor et à la position 6-inter alia par un groupe 3-aminopyrrolidino éventuellement protégé sont revendiqués comme intermédiaires de synthèse de 1,8-naphtyridines bactéricides dans le brevet BE-904086.

La demande de brevet japonais JP 62033176 (WPI 87-082820) décrit des dérivés de l'acide 3-nicotinoylacétique, substitués à la position 2- par un atome de chlore, à la position 5- par un atome de fluor et à la position 6- par, inter alia, un groupe amino cyclique éventuellement substitué, comme intermédiaires des naphtyridines, tandis que des dérivés de l'acide 2-pyridylaminométhylène-propandioïque, toujours utiles comme intermédiaires pour des naphtyridines, sont décrits dans EP-376870.

Enfin, la 3-amino-5-phényl-1-(2-pyridyl)pyrrolidine, possible intermédiaire d'antagonistes de l'histamine, ainsi que sa synthèse stéréosélective, ont été décrites dans J. Hétérocycl. Chem., 1973, **10** (5), 747-753.

On a maintenant trouvé que certains dérivés 1-hétéroaryl-azétidiniques et - pyrrolidiniques dans lesquels l'atome d'azote est lié à une pyridine, pyrazine ou pyrimidine éventuellement substituée et le carbone en position 3- porte un groupe aminométhyle ou amino éventuellement alkylé, ont une activité biochimique nouvelle très intéressante en se montrant des agonistes sélectifs des récepteurs 5-HT₃ à la sérotonine.

Plus particulièrement, donc, comme premier objet, la présente invention se réfère aux nouvelles 1-hétéroarylazétidines ou pyrrolidines répondant à la formule (I) suivante 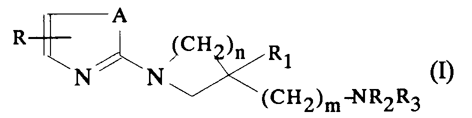
dans laquelle
- A: représente un groupement -CH=CH-, -CH=N- ou -N=CH-;
- R: représente un atome d'hydrogène, un atome d'halogène, un groupe (C₁-C₄)alkyle, (C₁-C₄)alcoxy, (C₁-C₄)alkylthio, un groupe cyano, carboxamido, trifluorométhyle, vinyle, formyle, un groupe carboxy libre, salifié ou esterifié, un groupe hydroxy, hydroxyméthyle, mercapto, un groupe amino, mono- ou di-(C₁-C₄)alkylamino, aminométhyle, mono- ou di-(C₁-C₄)alkylaminométhyle, 1-pipéridino, 1-pyrrolidino, 1-pipérazino ou 4-(C₁-C₄)alkyl-1-pipérazino, ce groupe R pouvant remplacer un quelconque des atomes d'hydrogène du noyau hétéroarylique;
- R₁: est un atome d'hydrogène ou un groupe méthyle;
- R₂ et R₃,: identiques ou différents, représentent un atome d'hydrogène ou un groupe (C₁-C₄)alkyle;

n est 1 ou 2,
m est 0 ou 1
et m + n ≧ 2 ;
lesdits composés pouvant être sous toutes les formes isomères éventuellement possibles, racémiques, énantiomères et diastéréoisomères, ainsi que sous forme de sels d'addition avec des acides minéraux ou organiques.

Les composés de formule (I) tels que définis ci-dessus ainsi que leurs sels d'addition avec les acides pharmaceutiquement acceptables présentent d'intéressantes propriétés pharmacologiques. Plus particulièrement ces produits sont doués de propriétés agonistes sélectives pour les récepteurs 5-HT₃ à la sérotonine. Ces propriétés justifient leur application en thérapeutique pour la préparation de médicaments destinés à la prophylaxie et au traitement des troubles du système sérotoninergique lorsque l'on souhaite avoir une action agoniste sélective médiée par les récepteurs 5-HT₃.

Dans les produits de formule (I) et dans ce qui suit:
- le terme "(C₁-C₄)alkyle" désigne un radical alkyle linéaire ou ramifié, contenant de 1 à 4 atomes de carbone, de préférence les radicaux méthyle, éthyle, n-propyle et isopropyle, mais également les radicaux n-butyle, isobutyle, sec-butyle et tert-butyle;
- le terme "(C₁-C₄)alcoxy" désigne un radical alcoxy à chaîne linéaire ou ramifiée, contenant de 1 à 4 atomes de carbone, de préférence les radicaux méthoxy et éthoxy, mais également les radicaux propoxy, isopropoxy, butoxy lineaire, secondaire ou tertiaire;
- le terme "(C₁-C₄)alkylthio" désigne un radical alkylthio à chaîne linéaire ou ramifiée, contenant de 1 à 4 atomes de carbone et de préférence les radicaux méthylthio, éthylthio et isopropylthio;
- le terme "atome d'halogène" désigne de préférence l'atome de chlore ou de brome, mais peut aussi représenter un atome de fluor ou d'iode;
- le terme "carboxy estérifié" désigne de préférence un groupe alcoxy inférieur carbonyle tel que méthoxycarbonyle, éthoxycarbonyle et tert-butoxycarbonyle, ou un groupe benzyloxycarbonyle, tandis que le terme "carboxy salifié" désigne de préférence un groupe carboxy salifié par un base minérale telle que par exemple un équivalent de sodium, de potassium, de calcium, de magnésium ou d'ammonium ou une base organique telle que par exemple la méthylamine, la propylamine, la triméthylamine, la N,N-diméthyléthanolamine, le tris (hydroxyméthyl)aminométhane, la pyridine, la picoline, la dicyclohexylamine, la benzylamine, la procaïne, la lysine, l'arginine, la N-méthylglucamine ou un composé de formule (I).

Les sels d'addition des composés de formule (I) avec les acides minéraux ou organiques peuvent être, par exemple, les sels formés avec les acides chlorhydrique, bromhydrique, iodhydrique, nitrique, sulfurique, oxalique, phosphorique, propionique, acétique, formique, benzoïque, maléique, fumarique, succinique, tartrique, citrique, oxalique, glyoxylique, aspartique, ascorbique, les acides sulfoniques, tels que l'acide méthanesulfonique, éthanesulfonique, méthanedisulfonique, benzènesulfonique, etc. On préfère notamment les sels formés avec l'acide chlorhydrique.

Une classe préférée de composés de formule (I) comprend les composés de formule (Ia) qui correspondent à la formule (I) où n et m sont 1 : 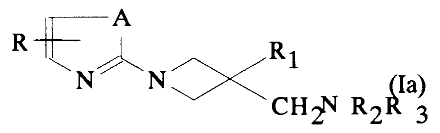
et R, A, R₁, R₂ et R₃ sont tels que définis ci-dessus.

Un groupe particulièrement préféré de composés de formule (Ia) selon la présente invention comprend les composés de formule (Ia) dans laquelle A représente un groupement -CH=CH- (dérivés pyridiniques) ou -N=CH- (dérivés pyraziniques) ; R, R₂ et R₃ sont tels que définis ci-dessus et R₁ est l'hydrogène ainsi que leurs sels d'addition acides.

Encore plus préférés sont les composés de formule (Ia) dans laquelle A, R₁, R₂ et R₃ sont tels que définis ci-dessus dans la définition d'un groupe préféré et R représente un atome d'hydrogène ou d'halogène, un groupe (C₁-C₄)alkyle, (C₁-C₄)alcoxy, cyano, carboxamido, trifluorométhyle, vinyle, formyle, carboxy libre, salifié ou esterifié, ou un groupe amino ainsi que leurs sels d'addition d'acides.

Particulièrement préférés sont les composés de formule (Ia) dans laquelle A représente un groupement -CH=CH-, R représente un atome de chlore ou brome aux positions 3-, 5- ou 6-, R₁ est un atome d'hydrogène et R₂ et R₃ sont identiques et représentent un atome d'hydrogène ou un groupe (C₁-C₄)alkyle, ainsi que leurs sels d'addition d'acides.

Une autre classe particulière de composés de formule (I) comprend les composés de formule (Ib) qui correspondent à la formule (I) où n est 2 : 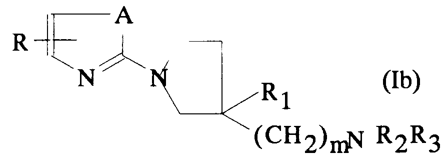
m est 0 ou 1 et tous les autres substituent R, A, R₁, R₂ et R₃ sont tels que définis ci-dessus.

Un groupe particulièrement préféré de composés de formule (Ib) selon la présente invention comprend les composés de formule (Ib) dans laquelle A représente un groupement -CH=CH- (dérivés pyridiniques) ou -N=CH- (dérivès pyraziniques), m, R, R₂ et R₃ sont tels que définis ci-dessus et R₁ est l'hydrogène ainsi que leurs sels d'addition acides.

Encore plus préférés sont les composés de formule (Ib) dans laquelle m, A, R₁, R₂ et R₃ sont tels que définis ci-dessus dans la définition d'un groupe préféré, et R représente un atome d'hydrogène ou d'halogène, un groupe (C₁-C₄)alkyle, (C₁-C₄)alcoxy, cyano, carboxamido,trifluorométhyle, vinyle, formyle, carboxy libre, salifié ou esterifié ou un groupe amino ainsi que leurs sels d'addition d'acides.

Particulièrement préférés sont les composés de formule (Ib) dans laquelle A représente un groupement -CH=CH-, R représente un atome de chlore ou brome aux positions 3-, 5- ou 6-, m est 0, R₁ est un atome d'hydrogène et R₂ et R₃, sont identiques et représentent un atome d'hydrogène ou un groupe (C₁-C₄)alkyle, ainsi que leurs sels d'addition d'acides.

Les composés de formule (I) de la présente invention peuvent être préparés à partir d'un composé hétérocyclique de formule (II) 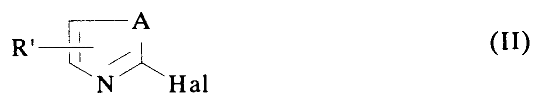
dans laquelle A a la signification donnée ci-dessus, Hal représente un atome d'halogène, le chlore, le brome et l'iode étant préférés et R' correspond à R ou à un groupe R protégé par un groupe protecteur approprié facilement éliminable; et d'un dérivé azétidinique ou pyrrolidinique de formule (III): 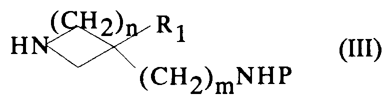
dans laquelle n, m et R₁ sont tels que définis ci-dessus et P représente un groupe protecteur temporaire du groupe amino convenablement choisi.

Les composés de départ de formule (II) sont en général des produits disponibles dans le commerce ou qui peuvent être préparés en laboratoire par des réactions très simples bien connues de l'homme du métier.

Les composés de départ de formule (III) où n est 1 peuvent être préparées à partir du 1-benzhydrylazétidin-3-ol ou du 1-benzhydryl-3-méthylazétidin-3-ol par conversion du groupe hydroxy en groupe mésyloxy, remplacement de ce dernier par un groupe cyano, réduction du groupe cyano à aminométhyl, protection du groupe amino par un groupe convenable P et élimination sélective du groupe benzhydryle par hydrogénation catalytique. Les pyrrolidines de départ de formule (III) où n est 2 et m est 0 peuvent être préparées à partir de la 1-benzyl-3-pyrrolidone (J. Org. Chem., 1965, **30**, 740). Lorsque R₁ est un atome d'hydrogène, la 1-benzyl-3-pyrrolidone est convertie en l'oxime correspondante par réaction avec l'hydroxylamine. La fonction oxime est ensuite réduite à l'aide d'un hydrure mixte tel que LiAlH₄, le groupe amino primaire qui se forme est protègé par l'introduction du groupe protecteur P et le groupe 1-benzyle est éliminé par hydrogénation catalytique avec des catalyseurs de platinum ou palladium.

Lorsque R₁ représente un groupe méthyle, on peut obtenir la 1-benzyl-3-méthyl-3-acétylaminopyrrolidine en suivant la méthode décrite dans EP-132845 (Exemple 7) et, éventuellement, avant d'éliminer le groupe benzyle par hydrogénation catalytique, en éliminant le groupe acétyle par hydrolyse et en introduisant un groupe protecteur P différent.

Les composés de formule (III) où n est 2 et m est 1 sont aisèment préparés à partir du 1-benzyl-3-pyrrolidinol qui est un produit commercial et du 1-benzyl-3-méthyl-3-pyrrolidinol (EP-132845-Exemple 7) par conversion dans le dérivé mésyle correspondant, remplacement du groupe mésyloxy par un groupe cyano et réduction du groupe cyano à aminométhyle à l'aide de LiAlH₄ suivi enfin de la déprotection par hydrogénation catalytique avec des catalyseurs de platinum. La réaction entre le composé de formule (II) et le dérivé de formule (III) conduit à un composé intermédiaire de formule (IV) 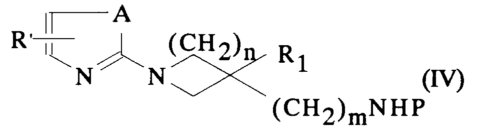
dans laquelle n, m, R', A, R₁ et P sont tels que définis ci-dessus, et l'élimination successive du ou des groupes protecteurs conduit au composé de formule (I) où n, m, R, A et R₁ sont tels que définis ci-dessus et R₂ et R₃ sont deux atomes d'hydrogène. Quand on veut obtenir un composé de formule (I) où R₂ et/ou R₃ sont différents de l'hydrogène, on poursuit par la mono- ou dialkylation du groupe amino selon les méthodes classiques connues.

Dans des conditions préférentielles de mise en oeuvre de l'invention, le procédé ci-dessus est réalisé de la manière suivante :

La réaction du composé de formule (II) avec le composé de formule (III) est conduite dans un solvant organique tel qu'un alcool, n-butanol, n-pentanol ou n-hexanol de préférence, dans le diméthylformamide, le diméthylsulfoxyde, le sulfolane, l' acétonitrile, la pyridine et similaires, à une température généralement comprise entre 50°C et 200°C, en présence d'un agent de condensation alcalin, tel qu'un hydroxyde, un carbonate ou un bicarbonate alcalin ou d'une amine tertiaire. Après 20 à 120 heures, la réaction est complète et le composé intermédiaire de formule (IV) ainsi obtenu est isolé selon les techniques habituelles.

On élimine ensuite le ou les groupes protecteurs dans les conditions usuelles connues de l'homme du métier et qui dépendent du groupe protecteur choisi.

Les groupes protecteurs de la fonction aminée préférés sont les groupes t-alcoxy-carbonyles tels que le t-butoxycarbonyle (BOC) ou le t-amyloxycarbonyle (AOC), le groupe BOC étant particulièrement préféré. L'élimination de ces groupes est donc aisement effectuée par hydrolyse acide selon des méthodes bien connues en littérature et notamment par action de l'acide trifluoroacétique ou de l'acide chlorhydrique dans un solvant alcoolique.

On peut aussi utiliser comme groupe protecteur de la fonction aminée le groupe acétyle qui peut être éliminé suivant les techniques d'hydrolyse acide ou basique bien connues par exemple en chauffant l'intermédiaire bloqué de formule (IV) dans une solution aqueuse d'un acide minéral ou organique, l'acide chlorhydrique de préférence, ou en le traitant avec une base minérale, hydroxyde de sodium de préférence.

Les diverses fonctions réactives éventuellement présentes dans certains composés de départ de formule (II) peuvent, si nécessaire ou convenable, être protégées: il s'agit par exemple des groupes hydroxy, amino, carboxy, et mercapto qui peuvent être protégés de façon conventionnelle. Comme exemple de protection du groupe hydroxy, on peut citer, de façon non exhaustive les radicaux silyles tels que le triméthylsilyle et le t-butyldiméthylsilyle et les groupes ethérifiants tels que le groupe tétrahydropyranyle.

Le substituant amino du noyau hétérocyclique peut être protégé comme indiqué plus haut par un groupe t-alcoxycarbonyle.

Le groupe carboxy peut être protégé sous forme d'ester facilement clivable tel qu'un ester benzylique ou t-butylique ou des esters connus dans la chimie des peptides. Il peut être aussi protégé sous forme d'alcool protégé correspondant et rétabli, ensuite, par oxydation.

Le groupe mercapto, enfin, peut être protégé par formation d'un disulfide, mixte ou symmétrique.

D'autres groupes protecteurs appropriés dans les conditions de réaction prévues ainsi que les méthodes pour les éliminer à la fin de la réaction sont décrits dans la littérature (voir par exemple D. Barton and W.C. Ollis, Comprehensive Organic Chemistry, vol. 5, pp. 323-331 et les références que l'on y trouve citées) et bien connus de l'homme du métier qui est donc en mesure de choisir les plus appropriés selon les circonstances.

On obtient ainsi un composé de formule (I) dans laquelle R₂ et R₃ sont tous les deux l'hydrogène. Ce composé peut donc être isolé et/ou transformé dans un de ses sels d'addition par traitement avec une solution hydroalcoolique ou organique de l'acide salifiant.

Si on veut obtenir un composé de formule (I) dans lequel l'un parmi R₂ et R₃ représente un groupe alkyle, on condense l'amine primaire ainsi obtenue avec l'aldéhyde ou la cétone convenablement choisie en formant une base de Schiff intermédiaire qui est réduite en utilisant les hydrides bien connus de l'homme de mètier, typiquement le cyanoborohydride de sodium.

On peut ensuite alkyler ultérieurement ce groupe amino secondaire selon les méthodes classiques d'alkylation des groupes amino, par exemple par réaction avec un halogénure alkylique, de préférence un iodure alkylique. On peut ainsi obtenir les dérivés dialkylés où R₂ et R₃ sont différents entre eux.

Quand on veut obtenir un composé de formule (I) où R₂ et R₃ sont identiques et représentent un groupe alkyle, on suit directement la méthode classique en utilisant au moins la quantité stoechiométriques d'agent alkylant.

Les composés de formule (Ib) de la présente invention contiennent, au moins, un atome de carbone chiral, celui qui porte le groupe -(CH₂)ₘ-NR₂R₃, et ils existent, donc, sous la forme de racémates ou d'énantiomères.

Les éniantiomères purs peuvent être obtenus soit en partant d'un composé de formule (III) sous forme optiquement active, soit par résolution du mélange racémique du composé (Ib) par exemple au moyen d'un agent de résolution tel qu'un dérivé de l'acide tartarique ou d'autres acides optiquement actifs.

Les composés de départ de formule (III), à leur tour, peuvent être obtenus, sous forme optiquement active par dédoublement des racémiques selon les méthodes usuelles connues de l'homme de métier, telles que par exemple la formation de sels diastéréoisomériques avec des acides optiquement actifs ou l'utilisation de colonnes chromatographiques chirales.

Si les substituants R, R₂ et R₃ contiennent des atomes chiraux additionnels, plusieurs formes isomèriques seront possibles, et les composés (Ib) pourraient être sous forme de racémates, énantiomères ou diastèréoisomères, formes isomériques, qui sont toutes comprises dans l'objet de la présente invention.

Les éventuelles formes optiquement actives des produits de formule (Ia) qui existent quand R₁ est un groupe méthyle et/ou les substituants R, R₂ et R₃ contiennent des atomes chiraux, peuvent être préparées par dédoublement des racémiques selon les méthodes usuelles connues de l'homme du métier, telles que par exemple la formation de sels diastéréoisomériques, ou l'utilisation de colonnes chromatographiques chirales.

Alternativement à la méthode générale décrite ci-dessus, ou de préférence, lorsque le composé de départ de formule (II) n'est pas un produit disponible dans le commerce, ou aisément préparable, on peut obtenir certains composés de formule (I) par modification du groupe R d'autres composés (I) ou de leurs intermédiaires (IV) selon des réactions bien connues dans la chimie classique.

Par exemple, quand on veut obtenir un composé de formule (I) où R est un groupe vinyle, on peut partir d'un composé (II) où R est un atome d'halogène, pour obtenir ainsi un composé intermédiaire (IV) où R est un atome d'halogène et remplacer alors cet atome par un groupe vinyle, par exemple par réaction avec un dérivé vinylique d'étain, tel que le tributylvinylétain, en présence de tétrakis(triphénylphosphine) palladium. On peut ensuite débloquer l'atome d'azote du groupe amino et l'alkyler éventuellement pour obtenir ainsi les composés (I) où A, R₁, R₂ et R₃ sont tels que définis ci-dessus et R est un groupe vinyle. Alternativement, avant de débloquer le groupe amino on peut oxyder le groupe vinylique, avec par exemple un periodate alcalin en présence de tétraoxyde d'osmium comme catalyseur, pour obtenir les composés correspondants où R est un groupe formyle.

A leur tour ces derniers composés peuvent être oxydés, par exemple avec le réactif de Jones, qui se compose d'une solution d'anhydride chromique dans de l'acide sulfurique, ou avec du chlorite alcalin, en donnant les acides carboxyliques correspondants. L'estérification de ces derniers, par exemple avec l'alcool correspondant en présence d'un agent déshydratant, conduit aux esters voulus.

De même, le radical carboxy estérifié peut être réduit en un radical hydroxyméthyle dans les conditions usuelles connues de l'homme du métier telles que notamment par action de l'hydrure de lithium et aluminium, de l'hydrure de diisobutylaluminium ou encore d'autres réducteurs connus de l'homme du métier.

L'éventuel substituant méthoxy des produits décrits ci-dessus peut être, si désiré, transformé en groupe hydroxy, par exemple par du tribromure de bore dans un solvant organique inerte tel que par exemple le chlorure de méthylène.

On peut aussi remplacer un atome d'halogène sur le noyau hétéroaromatique par un radical amino substitué, par exemple par traitement du composé halogéné avec une amine secondaire telle que les dialkylamines ou les amines cycliques, à la température ambiante dans un solvant tel qu'un alcool, notamment l'éthanol ou le méthanol.

De façon similaire, un atome d'halogène sur le noyau hétéroaromatique peut être converti dans un groupe alcoxy, e.g. méthoxy, par réaction avec un nucléophile tel qu'un alcoxyde, e.g. méthoxyde.

De même, cet atome d'halogène peut être converti en un groupe cyano par réaction par exemple avec le tributylcyanoétain en présence de tétrakis(triphénylphosphine) palladium. L'hydratation partielle des composés (I) ou (IV) où R est un groupe cyano, donne les composés correspondants où R est un groupe carboxamido. Cette hydrolyse du groupe cyano en groupe carboxamido peut être conduite dans des conditions contrôlées de catalyse acide ou, plus convenablement, dans des conditions basiques douces en présence de péroxyde d'hydrogène comme catalyseur.

A partir des composés intermédiaires de formule (IV), où R est un groupe cyano ou carboxamido, on peut ensuite obtenir les composés correspondants où R est un groupe aminométhyle par réduction avec un hydrure mixte tel que par exemple l'hydrure de lithium et d'aluminium et ses dérivés alcoxylés.

Le groupe amino peut ensuite être mono- ou di-alkylé selon les méthodes décrites précédemment.

D'autres réactions, bien connues dans la chimie classique peuvent être utilisées pour convertir un substituant R en un autre, soit dans les produits finals (I) soit dans les intermédiaires protégés (IV); toutes les méthodes de préparation des composés (I) faisant intervenir ces réactions tombent donc dans l'esprit de la présente invention.

Un objet ultérieur de la présente invention est donc un procédé de préparation d'un composé de formule (I) 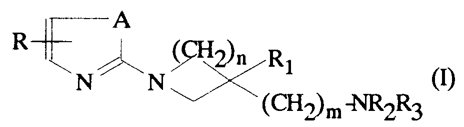
dans laquelle
- A: représente un groupement -CH=CH-, -CH=N- ou -N=CH-;
- R: représente un atome d'hydrogène, un atome d'halogène, un groupe (C₁-C₄)alkyle, (C₁-C₄)alcoxy, (C₁-C₄)alkylthio, un groupe cyano, carboxamido, trifluorométhyle, vinyle, formyle, un groupe carboxy libre, salifié ou esterifié, un groupe hydroxy, hydroxyméthyle, mercapto, un groupe amino, mono- ou di-(C₁-C₄)alkylamino, aminométhyle, mono- ou di-(C₁-C₄)alkylaminométhyle, 1-pipéridino, 1-pyrrolidino, 1-pipérazino ou 4-(C₁-C₄)alkyl-1-pipérazino, ce groupe R pouvant substituer un quelconque des atomes d'hydrogène du noyau hétéroarylique;
- R₁: est un atome d'hydrogène ou un groupe méthyle;
- R₂ et R₃,: identiques ou différentes, représentent un atome d'hydrogène ou un groupe (C₁-C₄)alkyle;

n est 1 ou 2; m est 0 ou 1 et n + m ≧ 2
caractérisé en ce que
on fait réagir un composé hétérocyclique halo-substitué de formule (II) 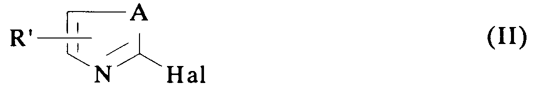
dans laquelle A a la signification donnée précédemment, R' correspond à R ou à un groupe R protégé par un groupe protecteur approprié facilement éliminable et Hal représente un atome d'halogène,
avec une azétidine ou pyrrolidine de formule (III) 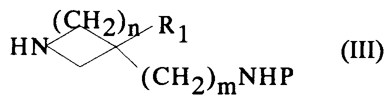
dans laquelle n, m, et R₁ sont tels que définis ci-dessus et P est un groupe protecteur temporaire du groupe amino convenablement choisi, pour obtenir un composé intermédiaire de formule (IV) 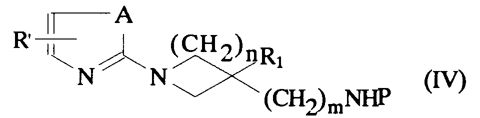
dans laquelle n, m, R', A, R₁ et P sont tels que définis ci-dessus,
- on élimine ensuite les groupes protecteurs présents, pour obtenir ainsi un composé de formule (I) où n, m, A, R et R₁ sont tels que définis ci-dessus et R₂ et R₃ sont l'hydrogène, et,
- quand on veut obtenir un composé de formule (I) dans laquelle R₂ et/ou R₃ sont différents de l'hydrogène, on alkyle l'atome d'azote selon des méthodes convenables; et,
- éventuellement on convertit le produit final en un de ses sels d'additions avec des acides minéraux ou organiques ; et,
- éventuellement après chaque étape, on peut convertir un substituant R en un autre selon des réactions connues en soi.

Les composés de formule (IV) où n, m, R', A, R₁ et P sont tels que définis ci-dessus sont des produits industriels nouveaux et des intermédiaires nécessaires à la préparation des composés de formule (I) et représentent donc un objet ultérieur spécifique de la présente invention.

Les composés de formule (I) tels que définis ci-dessus ainsi que leurs sels d'addition avec des acides pharmaceutiquement acceptables présentent d'intéressantes propriétés pharmacologiques.

Ces produits sont doués de propriétés agonistes sélectives pour le récepteur 5-HT₃ à la sérotonine.

L'affinité des composés de formule (I) pour les récepteurs 5-HT₃, a été mise en évidence à l'aide des essais de binding in vitro en utilisant les sites de liaison 5-HT₃ présents dans le cortex cérébral du rat (G.J. Kilpatrick, B.J. Jones et M.B. Tyers. Identification and distribution of 5-HT₃ receptors in rat brain using radioligand binding. Nature, 1987; **330:** 746-8) et comme ligand marqué le [³H] BRL 43694 (granisetron), un antagoniste des récepteurs 5-HT₃ puissant et spécifique.

La préparation des membranes et le test de binding ont été effectués selon la méthode décrite par Nelson et Thomas (D.R. Nelson et D.R. Thomas. [³H] BRL 43694 (granisetron), a specific ligand for 5HT₃ binding sites in rat brain cortical membranes. Biochem. Pharmacol., 1989; **38**: ₁693-5).

Les résultats ont été évalués avec des méthodes d'ajustement ("fitting") non linéaire "Accufit saturation", pour les études de saturation (H.A. Feldman. Mathematical theory of complex ligand-binding systems at equilibrium: some methods of parameter fitting. Analyt. Biochem., 1972; **48**: 317-38) et "Accufit competition", pour les études de déplacement (H.A. Feldman, D. Rodbard et D. levine. Mathematical theory of loss reactive radioimmunoassay and ligand-binding systems at equilibria. Analyt. Biochem., 1972; **45:** 530-56).

Afin d'obtenir les affinités des composés, dans les études de compétition on a utilisé une concentration de 0,5 nM de [³H] BRL 43694.

Dans ces essais in vitro les composés de formule (I) se sont montrés en général très puissants dans le déplacement du [³H] BRL 43694.

On a confirmé l'affinité et mis en évidence l'activité agoniste au niveau des récepteurs 5-HT₃ grâce aussi à des études chez le rat anésthésié, en particulier en administrant les composés par voie intraveineuse et en observant la diminution fugace de la fréquence cardiaque (effet Bezold-Jarisch), dont l'intensité varie selon la dose.

Cet effet est inhibé par les antagonistes sélectifs des récepteurs 5-HT₃ (par exemple ICS 205930 et zacopride), tandis qu'il n'est pas inhibé par les antagonistes des récepteurs D de la sérotonine (par exemple méthysergide).

Plus particulièrement, l'effet Bezold-Jarisch provoqué par les composés de formule (I) a été évalué en utilisant des rats Sprague-Dawley d'un poids compris entre 200 et 300 g, anésthésiés par une dose intrapéritonéale de 1,25 g/kg d'uréthane. La pression arterielle est enregistrée au niveau d'une artère carotide et la fréquence cardiaque évaluée par la fréquence des pulsations à l'aide d'un cardiotachymètre. Un cathéter est placé dans la veine jugulaire pour l'administration des substances.

Des doses différentes des composés à tester sont administrées par voie intraveineuse dans un volume de 0,5 ml/kg.

La bradycardie provoquée par chaque dose est exprimée comme inhibition en pourcentage de la fréquence basale. On peut ainsi calculer la DE₅₀, c'est-à-dire la dose qui diminue de 50% la fréquence cardiaque chez les animaux traités.

Les composés les plus intéressants ont montré des résultats, exprimés en DE₅₀, qui sont compris entre 0,5 et 10 µg/kg.

Ces propriétés justifient l'application de nouveaux composés de formule (I) selon l'invention en thérapeutique dans le traitement des troubles qui impliquent le système sérotoninergique soit périphérique soit central lorsque l'on souhaite avoir une action agoniste sélective mediée par les récepteurs 5-HT₃. On peut, par exemple, envisager l'emploi thérapeutique de ces produits dans le traitement des troubles dysthimiques, ou bien dans les cas d'anxiété ou de troubles psychotiques.

On peut également envisager l'utilisation de ces produits dans le traitement des désordres de la motricité intestinale et en particulier dans le traitement de la constipation.

L'invention a donc pour objet ultérieur l'utilisation des composés de formule (I) où n, m, R, A, R₁, R₂ et R₃ ont les significations données ci-dessus, ainsi que de leurs sels pharmaceutiquement acceptables, à titre de médicaments.

L'invention s'étend aux compositions pharmaceutiques renfermant à titre de principe actif l'un au moins des composés tels que définis ci-dessus.

Ces compositions pharmaceutiques peuvent être administrées par voie orale, rectale ou parentérale.

Ces compositions peuvent être solides ou liquides et se présenter sous toute forme pharmaceutique couramment utilisée en médecine humaine comme, par exemple, les comprimés simples ou dragéifiés, les gélules, les granulés, les solutions ou suspensions pour l'administration orale, les suppositoires et les préparations injectables; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs, etc.

La posologie usuelle, variable selon le produit spécifique employé, les conditions et le poids du sujet traité, l'affection en cause et le mode d'administration, peut être convenablement comprise entre 1 et 1000 mg par jour chez l'adulte.

Les exemples suivants illustrent l'invention sans toutefois la limiter.

### PREPARATION I

a) On chauffe à 60°C pendant 5 heures un mélange de 50 g (0,157 mol) de 1-benzhydryl-3-mésyloxyazétidine (J. Org. Chem. 1972, **37**, 3953-3955) et 23,5 g (0,479 mol) de cyanure de sodium dans 340 ml de diméthylformamide et 43 ml d'eau. On laisse le mélange réactionnel à température ambiante pendant une nuit et après on le verse dans 2000 ml d'eau/glace. On récupère le produit solide par filtration, on le purifie en le mettant en suspension dans 800 ml d'eau et en le filtrant. On sèche le produit ainsi obtenu et on le crystallise d'éther isopropylique pour obtenir 20 g de 1-benzhydryl-3-azétidine carbonitrile. P.f. 142-146°C.
b) On agite à température ambiante 0,37 g (0,008 mol) de l'hydrure de lithium et aluminium dans 40 ml d'éther éthylique anhydre et on y ajoute, par portions, pendant 10 minutes, 1 g (0,004 mol) du composé obtenu à l'étape a) ci-dessus. On chauffe au reflux pendant 2,5 heures, on refroidit et on dilue avec de l'eau. La phase organique est séparée, lavée à l'eau et séchée. Par addition d'isopropanol saturé de chlorure d'hydrogène on précipite le chlorhydrate de 3-aminométhyl-1-benzhydryl-azétidine (1,15 g). P.f. 202-204°C.
c) On ajoute lentement une solution de 7,5 ml (0,073 mol) d'anhydride acétique dans 10 ml d'acétate d'éthyle dans un mélange de 18 g (0,0713 mol) de 3-aminométhyl-1-benzhydryl-azétidine obtenue du chlorhydrate correspondant de l'étape b) par neutralisation avec de l'hydroxyde de sodium à 10%, extraction avec de l'acétate d'éthyle et évaporation du solvant, dans 150 ml d'acétate d'éthyle. On agite le mélange réactionnel à température ambiante pendant 1 h, on le refroidit et on y ajoute sous forte agitation 50 ml de NaOH 2N. Après 10 minutes sous agitation, on sépare la phase organique, on la lave à l'eau, on la sèche et on concentre sous pression réduite pour obtenir 20 g de 3-acétylaminométhyl-1-benzhydrylazétidine. P.f. 117-119°C.
d) On charge dans un appareil d'hydrogénation 19,5 g (0,0662 mol) du composé obtenu à l'étape c), 200 ml d'éthanol à 95%, 3,91 g de Pd(OH)₂ sur carbone à 20% et 5,4 ml d'acide chlorhydrique concentré. On met sous atmosphère d'hydrogène et on hydrogène à pression ambiante, à 40°C, pendant 3 heures. On filtre, on concentre le filtrat par évaporation sous pression réduite, on reprend le résidu huileux par du benzène (50 ml x 2) et ensuite par de l'acétone. On évapore le solvant organique sous pression réduite pour obtenir 10 g du chlorhydrate de 3-acétylaminométhylazétidine.

### PREPARATION II

a) On ajoute goutte à goutte une solution de 31 g (0,142 mol) de di-tert-butyldicarbonate dans 70 ml de chloroforme anhydre à une solution de 35,9 g (0,142 mol) de 3-aminométhyl-1-benzhydrylazétidine dans 360 ml de chloroforme anhydre sous atmosphère d'azote et on laisse sous agitation à température ambiante pendant 6 heures. On évapore le solvant et on reprend le résidu huileux par une petite quantité d'un mélange éther éthylique/hexane. On filtre et on sèche le produit solide ainsi obtenu à l'étuve pour obtenir 36,5 g de 1-benzhydryl-3-tert-butoxy-carbonylaminométhylazétidine. P.f. 108-110°C.
b) On chauffe à 50-60°C sous atmosphère d'hydrogène un mélange de 36,5 g (0,103 mol) de ce composé et 10 g de Pd(OH)₂ sur carbone à 20% dans 650 ml d'éthanol absolu. Après 4 heures, quand la quantité théorique d'hydrogène a été consommée, on élimine le catalyseur par filtration et on concentre le filtrat sous pression réduite. On reprend le produit solide ainsi obtenu par de l'éther isopropylique, on filtre et on sèche à l'étuve pour obtenir 19,8 g du 3-tert-butoxycarbonylaminométhylazétidine. P.f. 112-114°C.

### PREPARATION III

a) On dissout 10 g (0,0394 mol) de 1-benzhydryl-3-méthylazétidin-3-ol (S.S. Chattergee et A. Shoeb, Synthesis 1973, p. 153-154) dans 100 ml de pyridine anhydre, on refroidit le mélange à -20°C et on y ajoute goutte à goutte 7,3 g (0,063 mol) de chlorure de méthanesulfonyle. On maintient la température au dissous de - 10°C, on agite à cette température pendant 1 heure et on laisse le mélange réactionnel entre 0°C et 3°C pendant deux jours. On verse dans 600 ml d'eau/glace, on récupère le précipité par filtration et on le lave à l'eau. On sèche à l'étuve à 50°C pendant 2 heures, on reprend le produit dans de l'éther isopropylique (100 ml), on filtre et on sèche à l'étuve à 50°C pendant une nuit pour obtenir environ 10 g de 1-benzhydryl-3-méthanesulfonyloxy-3-méthylazétidine.
b) En opérant comme décrit dans la PREPARATION I, étapes a), b), c) et d), mais à partir du composé obtenu à l'étape a) ci-dessus, on obtient le chlorhydrate de 3-acétylaminométhyl-3-méthylazétidine.

### PREPARATION IV

a) On refroidit une solution de 2,6 g (0,025 mol) de diisopropylamine dans 80 ml de tétrahydrofuranne anhydre à -78°C sous azote et on y ajoute avec beaucoup de précaution 10 ml d'une solution 2M de nBuLi dans le n-hexane. On agite pendant 30 minutes et on y ajoute une solution de 6,2 g (0,025 mol) de 1-benzhydrylazétidin-3-carbonitrile, obtenu à l'étape a) de la PREPARATION I dans 30 ml de tétrahydrofuranne. Après 1 heure sous agitation on ajoute goutte à goutte une solution de 7,0 g (0,05 mol) de iodure de méthyle dans 10 ml de tétrahydrofuranne. On agite pendant 1 heure et on laisse le mélange réactionnel rechauffer jusqu'à la température ambiante. On laisse reposer pendant une nuit, on neutralise la solution basique par addition d'environ 100 ml d'une solution à 20% de NH₄Cl et quelques gouttes de HCl concentré. On extrait par du chlorure de méthylène et on concentre la phase organique pour obtenir 6,0 g de 1-benzhydryl-3-méthyl-3-azétidinecarbonitrile qu'on cristallise dans le n-hexane. P.f. 78-80°C.
b) On ajoute, par portions, 4,07 g (0,015 mol) du composé obtenu à l'étape a) ci-dessus à une suspension de 0,85 g (0,0225 mol) de LiAlH₄ dans 180 ml d'éther éthylique en contrôlant la température. On chauffe au reflux pendant 2,5 heures, on refroidit dans un bain de glace et on y ajoute environ 170 ml d'eau. On filtre, on lave le filtrat à l'eau, on le sèche sur sulfate de sodium et on concentre à pression réduite pour obtenir 2,7 g de 3-aminométhyl-1-benzhydryl-3-méthylazétidine sous forme de huile semi-solide jaune. Le sel d'addition correspondant avec l'acide oxalique, cristallisé dans l'acétone, a un p.f. 168-170°C.
c) On ajoute goutte à goutte une solution de 2,16 g (0,0096 mol) de ditert-butyldicarbonate dans 15 ml de chloroforme à une solution de 2,57 g (0,0096 mol) de 3-aminométhyl-1-benzhydryl-3-méthylazétidine dans 30 ml de chloroforme anhydre sous atmosphère d'azote. On agite à température ambiante, sous azote, pendant une nuit, on évapore à sec pour obtenir 2,7 g de 1-benzhydryl-3-tert-butoxycarbonylaminométhyl-3-méthylazétidine. P.f. 106-107°C.
d) On hydrogène, à pression atmosphérique et 50-60°C, un mélange de 4,2 g (0,0115 mol) du composé obtenu à l'étape précédente, 0,5 g de Pd(OH)₂ sur carbone à 20%, 70 ml d'éthanol absolu et 4 ml d'éthanol saturé de chlorure d'hydrogène. Quand la quantité théorique d'hydrogène a été consommée, on filtre et on concentre le filtrat à sec. On traite le résidu par 10 ml d'acétone, on le triture et on filtre pour obtenir 1,5 g du 3-tert-butoxycarbonylaminométhyl-3-méthylazétidine. P.f. 189-191°C.

### PREPARATION V

### a) 1-benzyl-3-cyanopyrrolidine

On ajoute goutte à goutte 4,8 g (0,004 mol) de chlorure de méthanesulfonyle à une solution de 5 g (0,028 mol) de 1-benzyl-3-pyrrolidinol dans 65 ml de pyridine anhydre refroidie à - 20°C. On agite pendant 1 heure, on laisse monter la température à la valeur ambiante et on continue l'agitation pendant 5 heures. On verse sur de la glace et on extrait au chlorure de méthylène. On évapore la phase organique sous vide en obtenant 7,9 g d'une huile brute qui est purifiée par chromatographie en éluant avec un mélange CH₂Cl₂/MeOH 98/2. On obtient 4,2 g (0,0166 mol) du mésylate qui sont dissous dans 25 ml de diméthylformamide. A la solution ainsi obtenue, on y ajoute une solution de 3,2 g (0,05 mol) de cyanure de potassium dans 6 ml d'eau et on chauffe à environ 70° C pendant 8 heures. Après une nuit de repos, on verse sur de la glace et on extrait à l'acétate d'éthyle. On lave la phase organique à l'eau, on la sèche sur Na₂SO₄ et on la concentre sous vide en obtenant 2,6 g d'un produit huileux qui est purifié par chromatographie sur colonne (éluant : cyclohexane/acétate d'éthyle 7/3). On obtient 1,3 g du composé indiqué dans le titre. P.e. = 100°C/0,3 mmHg.

### b) 1-benzyl-3-aminométhyl-pyrrolidine

On ajoute 3,49 g (0,019 ml) du composé obtenu à l'étape a) ci-dessus à une solution de 1,07 g (0,028 mol) de LiAlH₄ dans 140 ml d'éther éthylique anhydre sous agitation, de façon à garder une température constante. On chauffe à reflux pendant 3 heures, on refroidit et on détruit l'excès de LiAlH₄ en y ajoutant très lentement 200 ml d'eau. On filtre et on sépare la phase organique. On extrait la phase aqueuse au chlorure de méthylène et on ajoute cet extrait à la phase organique. On sèche sur Na₂SO₄ et on évapore sous vide en obtenant 3,48 g de 1-benzyl-3-aminométhyl-pyrrolidine.

### c) 1-benzyl-3-(tertbutoxycarbonylaminométhyl)pyrrolidine

On ajoute très lentement à une solution de 3,4 g (0,0179 mol) de 1-benzyl-3-aminométhylpyrrolidine dans 34 ml de CHCl₃ anhydre sous atmosphère d'azote et sous agitation, une solution de 3,9 g (0,0179 mol) de di-tertbutyl dicarbonate dans 8 ml de CHCl₃. On laisse sous azote et sous agitation pendant une nuit à température ambiante. On évapore le solvant sous vide et on obtient 5,5 g d'une huile qui est purifiée par chromatographie sur colonne en éluant avec un mélange CH₂Cl₂/MeOH 98/2. Rendement : 2,5 g (48 %).

### d) 3-(tertbutoxycarbonylaminométhyl)pyrrolidine

On dissout 2,1 g (0,0072 mol) du composé obtenu à l'étape c) ci-dessus dans 30 ml d'éthanol absolu. On y ajoute 0,3 g de Pd(OH)₂ sur charbon (catalyseur de Pearlman) et on hydrogène à 45° C et pression atmosphérique pendant 5 heures.

On filtre et on concentre sous vide en obtenant 1,5 g de 3-(tertbutoxycarbonylaminométhyl)pyrrolidine.

### PREPARATION VI

### a) Chlorhydrate de 1-benzyl-3-pyrrolidinone oxime.

On ajoute goutte à goutte une solution de 9,7 g (0,139 mol) de chlorhydrate d'hydroxylamine dans 30 ml d'eau à une solution de 25 g (0,19 mol) de 1-benzyl-3-pyrrolidinone dans 30 ml d'éthanol. On laisse à température ambiante pendant 30 minutes, on chauffe à 35°C pendant 30 minutes et on concentre sous pression réduite à 50°C. On cristallise d'alcool isopropylique le produit solide ainsi obtenu. P.f. 103-108°C. Rendement: 64,5 %.

### b) 1-Benzyl-3-pyrrolidineamine.

On prépare une solution de 7,3 g (0,192 mol) de LiAlH₄ dans 300 ml d'éther éthylique anhydre et on y ajoute, par portions, à température ambiante, 22 g (0,096 mol) du composé obtenu à l'étape a) ci-dessus.

On laisse à température ambiante pendant 1 heure et on chauffe au reflux pendant 3,5 heures. On détruit l'excès de LiAlH₄ et on filtre. On extrait le filtrat par 150 ml d'une solution 1N d'acide chlorhydrique. On rend la solution basique (pH 12) et on l'extrait par de l'éther éthylique. On sèche la phase organique et on la concentre pour obtenir 11 g du produit indiqué ci-dessus.

### c) 3-acétylamino-1-benzylpyrrolidine.

On ajoute goutte à goutte une solution de 16 g (0,178 mol) d'anhydride acétique dans 25 ml d'acétate d'éthyle dans une solution de 24 g (0,13 mol) du composé obtenu à l'étape précédente dans 125 ml d'acétate d'éthyle et on agite pendant 30 minutes jusqu'à la fin de la réaction. On rend basique par addition de 134 ml d'une solution 2N de NaOH, on sépare la phase organique, on extrait la phase aqueuse par 50 ml d'acétate d'éthyle, on sèche, on concentre et on y ajoute 150 ml de cyclohexane pour obtenir 11,3 g de 3-acétylamino-1-benzylpyrrolidine. P.f. 83-85°C.

### d) 3-acétylaminopyrrolidine

On hydrogène à 40°C un mélange de 12,5 g (0,057 mol) du composé obtenu à l'étape c) ci-dessus, 1,3 g de Pd/C à 10%, 80 ml d'éthanol à 95% et une goutte d'acide chlorhydrique concentré. Quand la quantité théorique d'hydrogène est consommée, on filtre et on concentre le filtrat pour obtenir 6,5 g du composé du titre sous forme d'un produit huileux.

### PREPARATION VII

### 3-tertbutoxycarbonylaminopyrrolidine.

On suit essentiellement la procédure de la PREPARATION VI en remplaçant dans l'étape c) l'anhydride acétique par du di-tertbutyldicarbonate et l'acétate d'éthyle par du chloroforme.

### PREPARATION VIII

### a) acide 6-chloro-2-pyridinecarboxylique

On chauffe à 90°C pendant 6 heures un mélange de 3,8 g (0,03 mol) de 6-chloro-2-méthylpyridine et 10,45 g de KMnO₄ dans 380 ml d'eau. On refroidit, on porte le pH à environ 4, on évapore l'eau et on reprend le résidu avec de l'éthanol. On filtre et on évapore l'éthanol en obtenant 1,1 g du composé indiqué ci-dessus.

### b) ester éthylique de l'acide 6-chloro-2-pyridinecarboxylique

On chauffe à reflux pendant 3 heures le composé obtenu à l'étape a) ci-dessus dans 10 ml d'éthanol saturé d'HCl.

On évapore l'éthanol, on reprend le résidu par de l'acétate d'éthyle, on lave à une solution de bicarbonate de sodium et après à l'eau. On sèche sur Na₂SO₄ et on évapore sous vide en obtenant 0,7 g de l'ester voulu.

### EXEMPLE 1

*Chlorhydrate de 2-(3-aminométhylazétidin-1-yl)-6-chloropyridine*.
a) On chauffe à la température de reflux pendant 5 heures un mélange de 6,17 g (0,0375 mol) du composé obtenu dans la PREPARATION I, 5,54 g (0,0371 mol) de 2,6-dichloropyridine et 13 g (0,094 mol) de carbonate de potassium anhydre dans 90 ml d'alcool n-amylique. On refroidit, on filtre et on concentre le filtrat à sec. On triture le résidu dans de l'eau (50 ml), on filtre, on lave le résidu sur filtre à l'eau, on le reprend dans de l'éther isopropylique, on évapore à sec et on cristallise dans 60 ml d'acétate d'éthyle pour obtenir 6 g de 2-(3-acétylaminométhylazétidin-1-yl)-6-chloro-pyridine. P.f. 136-138°C.
b) On chauffe au reflux pendant 24 h un mélange de 9 g (0,375 mol) du composé obtenu à l'étape a) ci-dessus et 35,7 g (0,60 mol) d'hydroxyde de potassium en poudre, dans 36,6 ml d'eau et 183 ml d'éthanol à 95%. On évapore jusqu'à un petit volume, on extrait à l'éther éthylique, on évapore l'éther et on dissout le résidu dans un mélange de 30 ml d'alcool isopropylique et 20 ml d'éther éthylique. On y ajoute de l'isopropanol saturé de chlorure d'hydrogène et on récupère le chlorhydrate (4,2 g) par filtration. P.f. 200-202°C.

### EXEMPLE 2

*Dichlorhydrate de 2-(3-aminométhylazétidin-1-yl)-6-chloropyrazine.*
a) On chauffe à la température de reflux pendant 48 heures un mélange de 2,97 g (0,02 mol) de 2,6-dichloropyrazine, 3,72 g (0,02 mol) du composé de la PREPARATION II sous forme de base libre et 2,02 g (0,02 mol) de triéthylamine dans 60 ml de toluène. On filtre sous vide à chaud et on évapore le solvant.
b) On fait réagir à température ambiante pendant une nuit le produit brut ainsi obtenu (4 g) avec 25 ml d'une solution d'acide chlorhydrique en éthanol. On sépare le précipité par filtration, on le lave avec de l'éthanol pour obtenir un produit solide jaune (2,7 g) cristallisé d'éthanol par addition de quelques gouttes d'eau. P.f. 133-135°C.

### EXEMPLE 3

*Dichlorhydrate de 2-(3-aminométhylazétidin-1-yl)-4-chloropyrimidine*

En suivant essentiellement la procédure décrite à l'Exemple 2, mais en partant de la 2,4-dichloropyrimidine au lieu de la 2,6-dichloropyrazine, on obtient le composé indiqué dans le titre. P.f. >300°C. Rendement 76%.

### EXEMPLE 4

*Oxalate de 2-(3-diméthylaminométhylazétidin-1-yl)-6-chloropyridine*

On dissout 0,5 g (0,0025 mol) du composé de l'Exemple 1 sous forme de base libre dans 0,5 ml d'acide formique à 85% (0,013 mol). On y ajoute 0,6 ml d'aldéhyde formique à 38% (0,08 mol) et on chauffe à 100°C pendant 1,5 heure. On porte à pH basique par addition d'une solution de NaOH, on extrait à l'acétate d'éthyle, on sèche les extraits organiques combinés sur Na₂SO₄ et on concentre sous pression réduite pour obtenir une huile jaune (0,45 g). On dissout cette huile dans 3 ml d'isopropanol et on y ajoute une solution de 0,25 g d'acide oxalique dans 1 ml d'isopropanol. On chauffe à la température de reflux jusqu'à solution et on laisse refroidir, pour récupérer 0,4 g du composé indiqué dans le titre ainsi précipité. P.f. 85-90°C.

### EXEMPLE 5

*Trichlorhydrate de 2-(3-aminométhylazétidin-1-yl)-6-bromopyridine*
a) On chauffe un mélange de 8,9 g (0,04 mol) de 3-tertbutoxycarbonylaminométhyl-azétidine, 9,47 g (0,04 mol) de 2,6-dibromopyridine et 13,8 g (0,1 mol) de carbonate de potassium anhydre dans 100 ml de diméthylsulfoxyde, à 110 °C pendant 3 heures. On verse dans l'eau, on extrait avec de l'acétate d'éthyle, on lave les extraits organiques combinés à l'eau, on sèche et on évapore à sec pour obtenir 13 g d'une huile jaune qui est purifiée par chromatographie flash sur colonne de gel de silice en éluant avec acétate d'éthyle/cyclohexane 2/8. Rendement de 2-(3-tertbutoxycarbonylaminométhylazétidin-1-yl)-6-bromopyridine: 4,5 g. P.f. 138-141°C (hexane).
b) On traite 1 g de ce dernier produit (0,029 mol) avec 10 ml d'éthanol saturé de chlorure d'hydrogène, sous agitation à température ambiante, pendant 4 heures. On évapore sous vide, on reprend par de l'isopropanol et on filtre pour obtenir 0,85 g du composé indiqué dans le titre. P.f. 185-187°C.

### EXEMPLE 6

*Oxalate de 2-(3-aminométhylazétidin-1-yl)-3-chloropyridine*
a) En suivant la procédure décrite à l'étape a) de l'Exemple 1, mais en utilisant la 2,3- dichloropyridine au lieu de la 2,6-dichloropyridine on obtient la 2-(3-acétylaminométhylazétidin-1-yl)-3-chloropyridine. P.f. 109-111°C.
b) On chauffe au reflux pendant 10 heures une solution de 0,6 g (0,025 mol) du composé obtenu ci-dessus dans 3 ml de HCl 6N. On évapore à pression réduite et on élimine l'eau en ajoutant de l'éthanol absolu et en l'évaporant, deux fois. On reprend avec une très petite quantité d'eau et on rend basique par addition d'une solution de NaOH. On extrait à l'acétate d'éthyle, on sèche les extraits organiques combinés sur Na₂SO₄ et on évapore à pression réduite, pour obtenir 0,6 g d'une huile jaune qui est dissoute dans 4 ml d'éthanol et acidifiée par addition d'une solution d'éthanol saturé d'acide oxalique. Par filtration on obtient 0,35 g du composé indiqué dans le titre. P.f. 188-191°C.

### EXEMPLE 7

*Chlorhydrate de 2-(3-aminométhylazétidin-1-yl)-5-chloropyridine*

On chauffe sous agitation à 100°C pendant 4,5 heures un mélange de 0,85 g (0,0038 mol) du composé de la PREPARATION II, 0,56 g (0,0038 mol) de 2,5-dichloropyridine et 1,3 g (0,0095 mol) de K₂CO₃ anhydre dans 10 ml de diméthylsulfoxyde. On verse le mélange dans l'eau, on extrait deux fois avec de l'acétate d'éthyle et on concentre à pression réduite pour obtenir un produit huileux qui est purifié par chromatographie sur colonne de gel de silice en éluant avec un mélange acétate d'éthyle/cyclohexane 1/1. On obtient ainsi 0,32 g d'une huile semi-solide qui est dissoute dans 3 ml d'éthanol saturé de chlorure d'hydrogène. Après 3 heures d'agitation à température ambiante on filtre le précipité, on le lave à l'éthanol d'abord et à l'éther éthylique ensuite et on le sèche pour obtenir 0,2 g du composé indiqué dans le titre. P.f. 290-295°C (déc.).

### EXEMPLE 8

*Chlorhydrate de 2-(3-aminométhylazétidin-1-yl)-6-bromopyridine*

On obtient ce composé (le même que celui de l'Exemple 5) en partant aussi de la 3-acétylaminométhylazétidine, en suivant la procédure de l'Exemple 1a) et en soumettant la 2-(3-acétylaminométhylazétidin-1-yl)-6-bromopyridine ainsi obtenue (P.f. 115-117°C) à une hydrolyse acide en suivant essentiellement la procédure décrite à l'Exemple 1b).

### EXEMPLE 9

*Maléate de 2-(3-aminométhyl-3-méthylazétidin-1-yl)-6-chloropyridine*
a) On chauffe au reflux pendant 6 heures un mélange de 1,36 g (0,0057 mol) du composé de la PREPARATION IV, 0,85 g (0,0057 mol) de 2,6-dichloropyridine et 2,0 g (0,014 mol) de K₂CO₃ dans 15 ml d'alcool n-pentylique. On refroidit, on élimine les sels par filtration et on évapore sous pression réduite pour obtenir une huile jaune qui est purifiée par chromatographie sur colonne de gel de silice en éluant avec un mélange chlorure de méthylène/méthanol 95/5. On obtient ainsi 750 mg de 2-(3-tertbutoxycarbonylaminométhyl-3-méthylazétidin-1-yl)-6-chloropyridine. P.f. 108-111°C.
b) On agite pendant une nuit une solution de 0,7 g (0,0022 mol) du composé obtenu à l'étape a) ci-dessus, dans 10 ml d'éthanol saturé de chlorure d'hydrogène. On évapore l'éthanol, on reprend le résidu huileux avec une solution à 10% de Na₂CO₃ et on extrait à l'acétate d'éthyle. Par évaporation à pression réduite on obtient 400 mg d'un produit huileux jaune qui est dissout dans une petite quantité d'éthanol et précipité sous forme de maléate par addition d'une solution d'acide maléique dans l'éthanol. P.f. 164-166°C. Rendement: 400 mg.

### EXEMPLE 10

*2-(3-méthylaminométhylazétidin-1-yl)-6-chloropyridine*
a) On chauffe à 100°C pendant 4,5 heures un mélange de 0,56 g (0,0038 mol) de 2,6-dichloropyridine, 0,85 g (0,0038 mol) du composé de la PREPARATION II et 1,3 g (0,0095 mol) de K₂CO₃ anhydre broyé dans 10 ml de diméthylsulfoxyde. On verse dans l'eau et on extrait à l'acétate d'éthyle. On évapore à sec et on purifie le résidu par chromatographie sur colonne de gel de silice en éluant avec un mélange cyclohexane/acétate d'éthyle 7/3. Par évaporation des fractions le contenant on obtient 0,33 g de 2-(3-tertbutoxycarbonylaminométhylazétidin-1-yl)-6-chloropyridine. P.f. 124-126°C.
b) On refroidit à une température comprise entre 0 et 5°C une solution de 0,31 g (0,001 mol) du composé obtenu à l'étape a) ci-dessus dans 1 ml de tétrahydrofuranne et on y ajoute 3 ml d'une solution 1M de complexe borane-tétrahydrofuranne. On chauffe à reflux pendant une nuit, on refroidit, on y ajoute 2 ml de méthanol et on chauffe à reflux pendant 1 heure; on refroidit encore, on ajoute 3 ml d'une solution d'HCl et on chauffe à reflux pendant 2 heures. On évapore à sec sous vide, on reprend le résidu par de l'éthanol absolu et on évapore à sec sous vide. On repète cette opération et ensuite on chromatographie le résidu ainsi obtenu sur une colonne de gel de silice en éluant avec un mélange acétate d'éthyle/méthanol 7/3. On récupère 0,15 g du composé indiqué dans le titre sous forme d'une huile jaune.

### EXEMPLE 11

*Oxalate de 2-(3-méthylaminométhylazétidin-1-yl)-6-chloropyridine*

On prépare le composé de l'exemple 10, sous forme d'oxalate, selon la méthode alternative suivante :
a) On chauffe à 85° C pendant 6 heures un mélange de 1,5 g (0,0076 mol) de 2-(3-aminométhylazétidin-1-yl)-6-chloropyridine (le composé de l'Exemple 1 sous forme de base libre) et 1,1 g (0,0152 mol) de formiate d'éthyle. On laisse au repos pendant une nuit, on traite par de l'hexane et on décante. On évapore le résidu à sec en obtenant une huile semisolide (1,15 g) correspondant au dérivé N-formyle.
b) On ajoute très lentement une solution 1M de BH₃ THF (12 ml) à une solution de 1,1 g (0,0049 mol) du composé obtenu à l'étape a) ci-dessus dans 4 ml de tètrahydrofuran anhydre sous atmosphère d'azote et refroidie à 0° C. On chauffe à reflux pendant 4 heures, on refroidit à 0° C et on y ajoute, goutte à goutte très lentement, 6 ml de méthanol. On chauffe à reflux pendant une demi-heure, on refroidit, on y ajoute 5 ml d'HCl 5N et on chauffe encore à reflux pendant 90 minutes. On évapore le solvant organique, on lave la phase aqueuse à I'acétate d'éthyle et on la rend basique par addition d'une solution concentrée de NaOH. On extrait par de l'acètate d'éthyle, on lave l'extrait organique avec une très petite quantité d'eau, on sèche sur Na₂SO₄ et on évapore sous vide.

On dissout le produit huileux dans une petite quantité de isopropanol et on y ajoute un excès d'acide oxalique. Après quelques jours à 2-4°C, on sépare le composé indiqué dans le titre par filtration. P.f. 160-170°C.

### EXEMPLE 12

*Chlorhydrate de 6-[(3-aminométhyl)azétidin-1-yl]-2-pyridinecarbonitrile*

### a) 6-[(3-tertbutoxycarbonylaminométhyl)azétidin-1-yl]-2-pyridinecarbonitrile

On chauffe au reflux pendant 4 heures une mélange de 0,2 g (0,00058 mol) de 2-bromo-6-(3-tertbutoxycarbonylaminométhyl)azétidin-1-ylpyridine obtenue à l'étape a) de l'Exemple 5, 5 ml de dioxanne anhydre, 0,68 g (0,00058 mol) de tetrakis(triphénylphosphine)palladium, 0,22 g (0,0007 mol) de cyanure de tributylétain, 0,07 g (0,0017 mol) de LiCl anhydre et quelques cristaux de 2-tertbutyl-4-méthylphénol. On refroidit et on y ajoute goutte à goutte 6 ml de pyridine et 20 ml d'une solution 1,1 M de fluorure de tétrabutylammonium dans du tétrahydrofuranne. Après une nuit à température ambiante on dilue avec de l'éther éthylique et on filtre sur célite. On concentre et on obtient un produit huileux qui est purifié par chromatographie en éluant avec un mélange cyclohéxane/acétate d'éthyle 7/3. On obtient 0,06 g du composé intermédiaire indiqué ci-dessus sous forme de produit solide fluorescent.

### b) Chlorhydrate de 6-[(3-aminométhyl)azétidin-1-yl]-2-pyridinecarbonitrile.

On agite à température ambiante pendant une nuit un mélange de 0,06 g du composé de l'étape a) ci-dessus et 0,3 ml d'acide trifluoroacétique dans 3 ml de chloroforme. On lave avec une solution saturé de NaHCO₃ et on extrait à l'acétate d'éthyle. On sèche la phase organique sur sulfate de sodium, et on concentre sous vide pour obtenir 0,2 g du composé indiqué dans le titre sous forme de huile. On le dissout dans une petite quantité d'isopropanol, on acidifie par addition d'isopropanol saturé de chlorure d'hydrogène et on récupère le précipité par filtration pour obtenir 20 mg du composé indiqué dans le titre. P.f. 275-280°C.

### EXEMPLE 13

*Chlorhydrate de 6-(3-aminométhylazétidin-1-yl)-2-aminopyridine*

### a) 2-tertbutoxycarbonylamino-6-chloro-pyridine.

On ajoute goutte à goutte une solution de 4,36 g (0,02 mol) de ditertbutyldicarbonate dans 10 ml de chlorure de méthylène à une solution de 2,57 g (0,02 mol) de 2-amino-6-chloropyridine dans 25 ml de chlorure de méthylène. On laisse à température ambiante pendant une nuit, on lave avec une solution 1N d'HCl et à l'eau, on sèche sur sulfate de sodium et on concentre sous vide pour obtenir 4,0 g du composé indiqué ci-dessus comme huile jaune.

### b) 6-[(3-tertbutoxycarbonylaminométhyl)azétidin-1-yl]-2-tertbutoxycarbonylaminopyridine.

On chauffe à 120°C ext. pendant une nuit un mélange de 1,7 g (0,0075 mol) du composé obtenu à l'étape a) ci-dessus, 1,4 g (0,0063 mol) de 3-tertbutoxycarbonylaminométhylazétidine (PREPARATION II) et 2,2 g (0,0157 mol) de K₂CO₃ dans 20 ml de diméthylsulfoxyde. On ajoute 20 ml d'eau et on extrait avec de l'acétate d'éthyle. On lave la phase organique à l'eau, on la sèche et on l'évapore sous vide. On purifie le résidu par chromatographie en éluant avec un mélange acétate d'éthyle/cyclohexane 1/9 pour obtenir 1 g du composé indiqué ci-dessus qui est lavé avec de l'hexane.

### c) Chlorhydrate de 6-(3-aminométhylazétidin-1-yl)-2-aminopyridine.

On agite à température ambiante pendant une nuit un mélange de 0,9 g du composé de l'étape b) ci-dessus et 15 ml d'éthanol saturé de chlorure d'hydrogène. On évapore à sec pour obtenir le composé indiqué dans le titre sous forme d'un produit huileux qui cristallise par traitement avec éthanol à 95%.

### EXEMPLE 14

*Chlorhydrate de 2-(3-aminométhylazétidin-1-yl)-6-trifluorométhylpyridine*

### a) 2-[(3-tertbutoxycarbonylaminométhylazétidin-1-yl)-6-trifluorométhylpyridine.

On chauffe à 120°C ext. sous agitation pendant une nuit un mélange de 1,55 g (0,007 mol) de 3-tertbutoxycarbonylaminométhylazétidine (PREPARATION II), 1,52 g (0,0084 mol) de 2-chloro-6-trifluorométhylpyridine et 2,41 g (0,0175 mol) de carbonate de potassium dans 20 ml de diméthylsulfoxyde. On ajoute 30 ml d'eau, on extrait à I'acétate d'éthyle, on lave la phase organique à l'eau, on la sèche et on l'évapore sous vide. On purifie le résidu par chromatographie en éluant avec un mélange cyclohexane/acétate d'éthyle 7/3 pour obtenir 0,95 g du composé intermédiaire indiqué ci-dessus sous forme de produit solide blanc.

### b) Chlorhydrate de 2-(3-aminométhylazétidin-1-yl)-6-trifluorométhylpyridine.

On agite à température ambiante pendant 3 heures une solution de 0,95 g du composé obtenu à l'étape précédente dans 12 ml d'éthanol saturé de chlorure d'hydrogène. On évapore l'éthanol, on reprend le résidu huileux ainsi obtenu par de l'isopropanol et on cristallise le produit du titre par réfrigération. On obtient 0,35 g. P.f. 149-151°C.

### EXEMPLE 15

*Chlorhydrate de 2-(3-aminométhylazétidin-1-yl)-6-méthoxypyridine*

### a) 2-[(3-tertbutoxycarbonylaminométhylazétidin-1-yl)-6-méthoxypyridine.

On suit essentiellement la procédure de l'étape a) de l'Exemple 16 en utilisant 1,2 g de 2-chloro-6-méthoxypyridine au lieu de la 2-chloro-6-trifluorométhylpyridine et on obtient le produit intermédiaire indiqué ci-dessus sous forme de huile.

### b) Chlorhydrate de 2-(3-aminométhylazétidin-1-yl)-6-méthoxypyridine.

On suit la procédure de l'étape b) de l'Exemple 16 mais à partir composé intermédiaire obtenu à l'étape a) ci-dessus et on obtient le composé indiqué dans le titre. P.f. 160-165°C.

### EXEMPLE 16

*Chlorhydrate de 2-(3-aminométhylazétidin-1-yl)-3-chloropyridine*

### a) 2- [(3-tertbutoxycarbonylaminométhylazétidin-1-yl)-3-chloropyridine.

On chauffe à 110°C ext. pendant 4,5 heures un mélange de 0,85 g (0,0038 mol) du composé de la PREPARATION II, 0,56 g (0,0038 mol) de 2,3-dichloropyridine et 1,3 g (0,0095 mol) de carbonate de potassium dans 10 ml de diméthylsulfoxyde. On verse dans l'eau et on extrait par de l'acétate d'éthyle. On sèche la phase organique sur sulfate de sodium, on la concentre sous vide et on purifie le résidu par chromatographie pour obtenir 0,7 g du produit intermédiaire indiqué ci-dessus.

### b) Chlorhydrate de 2-(3-aminométhylazétidin-1-yl)-3-chloropyridine.

On dissout le produit obtenu à l'étape a) dans 70 ml d'éthanol saturé de chlorure d'hydrogène. On agite à température ambiante pendant 3 heures, on évapore l'éthanol, on reprend par quelques millilitres d'isopropanol chaud, on refroidit et on filtre pour obtenir 0,45 g du composé indiqué dans le titre. P.f. 187-192°C.

### EXEMPLE 17

*Chlorhydrate de 2-(3-aminométhylazétidin-1-yl)-6-méthylpyridine*

### a) 2-[(3-tertbutoxycarbonylaminométhylazétidin-1-yl)-6-méthylpyridine.

On chauffe à 120°C pendant 6 heures un mélange de 2,9 g (0,013 mol) du composé de la PREPARATION II, 2,2 ml (0,0195 mol) de 2-chloro-6-méthylpyridine et 4,5 g (0,0325 mol) de carbonate de potassium anhydre dans 35 ml de diméthylsulfoxyde. On y ajoute 50 ml d'eau, on extrait par de l'acétate d'éthyle, on lave la phase organique à l'eau, on la sèche sur du sulfate de sodium et on évapore à pression réduite. On purifie le résidu huileux par chromatographie en éluant avec un mélange cyclohexane/acétate d'éthyle 7/3 pour obtenir 0,46 g du produit indiqué ci-dessus.

### b) Chlorhydrate de 2-(3-aminométhylazétidin-1-yl)-6-méthylpyridine.

On dissout 0,46 g (0,0016 mol) du composé obtenu à l'étape a) ci-dessus dans 4 ml d'éthanol saturé de chlorure d'hydrogène et on laisse à température ambiante pendant 3 heures. On y ajoute une petite quantité d'isopropanol et on filtre. On lave à l'éther éthylique le produit solide et on le sèche pour obtenir 350 mg du composé indiqué dans le titre. P.f. 300-310°C (déc.).

### EXEMPLE 18

*Oxalate de 6-[(3-aminométhyl)azétidin-1-yl]-2-pyridinecarbonitrile*

On prépare le composé de l'Exemple 10 sous forme d'oxalate par la méthode alternative suivante :

On chauffe à 80-85° C pendant une nuit un mélange de 7,3 g (0,052 mol) de 6-chloropyridine-2-carbonitrile (CAS 103 : 71164 n), 8,31 g (0,052 mol) du dichlorhydrate de la 3-aminométhylazétidine et 25 g de K₂CO₃ anhydre dans 130 ml de diméthylsulfoxyde.

On filtre, on évapore sous pression réduite à 70-80°C et on purifie le résidu par chromatographie sur colonne en éluant avec du méthanol. Par évaporation du solvant, on obtient 3,8 g de la base libre qui est dissoute dans une petite quantité d'acétone et précipitée sous forme d'oxalate par addition d'un excès d'acide oxalique. P.f. 185-190°C.

### EXEMPLE 19

*Chlorhydrate de l'ester éthylique de l'acide 6-(3-aminométhylazétidin-1-yl)-2-pyridine-carboxylique*
a) On chauffe à reflux pendant 3 heures un mélange de 3,15 g (0,0092 mol) du composé obtenu à l'étape a) de l'EXEMPLE 5, 3,7 g (0,00117 mol) de tributylvinylétain, 1,16 g (0,027 mol) de LiCl anhydre, 0,2 g (0,00018 mole) de tétrakis-(triphénylphosphine)palladium et un très petite quantité de 2-tertbutyl-4-méthylphénol dans 54 ml de dioxanne anhydre. On laisse refroidir et on y ajoute 6 ml de pyridine et 15 ml d'une solution 1,1 M de chlorure de tétrabutylammonium dans le tétrahydrofuranne. Après une nuit sous agitation, on ajoute 100 ml d'éther éthylique et on filtre sur célite. On évapore la solution organique à sec pour obtenir la 2-(3-tertbutoxycarbonylaminométhylazétidin-1-yl)-6-vinylpyridine sous forme d'une huile jaune qui est cristallisée dans l'hexane (2,4 g). P.f. 85-87°C.
b) On ajoute 6,8 g (0,031 mol) de périodate de sodium dans un mélange de 3,1 g (0,01 mol) du composé obtenu ci-dessus, 0,07 g ( 0,00028 mol) de tétraoxyde d'osmium, 80 ml de tétrahydrofuranne et 23 ml d'eau et on agite à la température ambiante pendant 3 heures. On verse dans 120 ml d'eau, on extrait à l'acétate d'éthyle, on lave la phase organique à l'eau, on la sèche sur sulfate de sodium, on filtre et on évapore à sec. On obtient une huile jaune (3 g) qui après cristallisation, par addition d'une petite quantité d'éther éthylique, donne 2,2 g de 6-(tertbutoxycarbonylaminométhylazétidin-1-yl)-2-pyridinecarboxyaldéhyde. P.f. 125-127°C.
c) On ajoute une solution de 10,3 g de NaClO₂ à 80% et 9,2 g de NaH₂PO₄ dans 55 ml d'eau distillée à une solution du composé obtenu à l'étape b) ci-dessus dans 150 ml de tertbutanol et on agite à température ambiante pendant 1 heure. On ajoute ensuite 120 ml d'eau et on extrait à l'acétate d'éthyle. On sèche l'extrait organique sur sulfate de sodium, on filtre et on évapore à sec pour obtenir une huile jaune correspondante à l'acide 6-(3-tertbutoxycarbonylaminométhylazétidin-1-yl)-2-pyridinecarboxylique qui est purifiée par chromatographie sur colonne en éluant avec un mélange acétate d'éthyle/méthanol 7/3.
d) On agite à température ambiante pendant une nuit un mélange de 1,9 g du composé obtenu à l'étape c) ci-dessus et 10 ml d'éthanol absolu saturé de chlorure d'hydrogène; ensuite on èvapore à sec, on ajoute 15 ml d'éthanol saturé de chlorure d'hydrogène et on chauffe au reflux pendant 3 heures. On évapore à sec, on reprend le résidu avec 50 ml d'acétate d'éthyle et 20 ml d'une solution aqueuse de bicarbonate de sodium, en sépare la phase organique qui est ensuite lavée à l'eau, séchée et évaporée. On reprend le résidu par 20 ml d'acétone, on acidifie la solution par addition d'isopropanol saturé de chlorure d'hydrogène et on récupère le produit cristallisé par filtration. P.f. 118-120°C.

### EXEMPLE 20

*Chlorhydrate de l'ester éthylique de l'acide* *6-(3-aminométhylazétidin-1-yl)-2-pyridinecarboxylique*

On obtient le composé de l'Exemple 19 aussi par la méthode alternative suivante :

On chauffe à 110° C pendant une nuit sous agitation un mélange de 0,65 g (0,0029 mol) du composé de la Préparation II sous forme de chlorhydrate, 0,53 g (0,0029 mol) du composé de la préparation VIII et 1,0 g (0,00725 mol) de K₂CO₃ anhydre dans 8 ml de diméthylsulfoxyde. On verse le mélange réactionnel dans de l'eau et on extrait deux fois avec de l'acétate d'éthyle. On concentre la phase organique sous vide et on purifie le résidu huileux ainsi obtenu par chromatographie en éluant avec un mélange cyclohexane/acétate d'éthyle 7/3. On dissout le produit ainsi obtenu dans 3 ml d'éthanol saturé d'HCl et on laisse sous agitation à température ambiante pendant une nuit. On y ajoute 30 ml d'éthanol absolu, on concentre sous pression réduite, on reprend le résidu avec de l'isopopranol et on laisse cristalliser le composé du titre qui est ensuite séparé par filtration. Rendement 0,16 g. P.f. 119-121°C.

### EXEMPLE 21

*6 -[(3-aminométhyl)azétidin-1-yl]-2-pyridinecarboxamide*

On chauffe à la température du reflux pendant 4 heures un mélange de 0,8 g (0,0042 mol) du composé de l'exemple 10, 4 ml d'eau distillée et 8 ml d'HCl 5 N, et on évapore à sec. On reprend le résidu avec de l'éthanol et on évapore l'éthanol, on répète cette opération plusieurs fois et enfin on reprend avec de l'isopropanol. Après une nuit à 2-4° C, on filtre en obtenant 0,95 g du composé indiqué dans le titre. P.f. 297-303° C. Par cristallisation d'éthanol à 90° C on obtient un produit avec p.f. >310° C

### EXEMPLE 22

*Chlorhydrate de 6-(3-aminométhylazétidin-1-yl)-2-vinylpyridine*

On dissout 0,5 g (0,0017 mol) du composé obtenu à l'étape a) de l'Exemple 19 dans 5 ml d'éthanol saturé de chlorure d'hydrogène et on agite à la température ambiante pendant 3 heures. On évapore l'éthanol, on reprend le résidu dans de l'isopropanol et on filtre pour obtenir 0,35 g du composé indiqué dans le titre sous forme de solide jaune. P.f. 285-286°C.

### EXEMPLE 23

*Chlorhydrate de 6-(3-aminométhylazétidin-1-yl)-2-pyridinecarboxaldéhyde*

On dissout 0,6 g (0,002 mol) du composé obtenu à l'étape b) de l'Exemple 19 dans 8 ml d'éthanol saturé de chlorure d'hydrogène et on agite à la température ambiante pendant 3 heures. On concentre sous vide et on obtient le produit indiqué dans le titre sous forme d'une huile foncée.

### EXEMPLE 24

*Chlorhydrate de 2-[(3-aminométhyl)azétidin-1-yl]-6-(1-piperidinyl)pyridine*

### a) 2-[(3-tertbutoxycarbonylaminométhyl)azétidin-1-yl]-6-(1-pipéridinyl)pyridine

On chauffe à environ 100° C pendant 8 heures un mélange de 0,3 mg (0,8 mmol) du composé obtenu à l'étape a) de l'Exemple 5, 0,1 ml (0,8 mmol) de pipéridine et 0,17 g (1,2 mmol) de K₂CO₃ dans 3 ml de diméthylsulfoxyde sous agitation. On verse le mélange réactionnel dans de l'eau et on extrait deux fois à l'acétate d'éthyle et une fois au chlorure de méthylène. On concentre sous pression réduite et on obtient 0,4 g d'un produit huileux qui cristallise spontanément.

### b) Chlorhydrate de 2-[(3-aminométhyl)azétidin-1-yl]-6-(1-pipéridinyl)pyridine

On dissout le produit obtenu à l'étape a) dans 3 ml d'éthanol saturé d'HCl, on laisse sous agitation à température ambiante pendant une nuit et on sépare le produit indiqué dans le titre par filtration.

### EXEMPLE 25

*Dichlorhydrate de* *2-(3-aminopyrrolidin-1-yl)-6-chloropyrazine*.

### a) 2-chloro-6-(3-tertbutoxycarbonylaminopyrrolidin-1-yl)pyrazine.

On chauffe à reflux pendant 24 heures un mélange de 2,24 g (0,012 mol) du composé obtenu dans la PREPARATION VII, 1.79 g (0,012 mol) de 2,6-dichloropyrazine et 1,2 g (0,012 mol) de triéthylamine dans 50 ml de toluène. On filtre à chaud pour éliminer les sels, on évapore le solvant et on purifie le produit huileux qu'on obtient par chromatographie sur colonne de gel de silice en éluant avec un mélange chlorure de méthylène/acétate d'éthyle 9/1. On obtient ainsi 600 mg du composé indiqué ci-dessus.

### b) Dichlorhydrate de 6-(3-aminopyrrolidin-1-yl)-2-chloropyrazine.

On dissout le produit de l'étape a) ci-dessus dans 10 ml d'éthanol saturé de chlorure d'hydrogène et on laisse sous agitation à température ambiante pendant une nuit. On filtre et on obtient 0,41 g du composé du titre sous forme d'un produit solide jaune qu'on recristallise dans l'éthanol. P.f. 233-235°C.

### EXEMPLE 26

*Dichlorhydrate de 2-(3-aminopyrrolidin-1-yl)-6-chloropyrimidine.*

### a) 6-chloro-2-(3-tertbutoxycarbonylaminopyrrolidin-1-yl)pyrimidine.

On chauffe à reflux pendant 48 heures un mélange de 2,77 g (0,015 mol) de 3-tertbutoxycarbonylaminopyrrolidine (PREPARATION VII), 2,21 g (0,015 mol) de 2,6-dichloropyrimidine et 1,5 g (0,013 mol) de triéthylamine dans 50 ml de toluène. On laisse refroidir, on filtre et on évapore le filtrat à sec pour obtenir un produit brut qui est purifié par chromatographie sur colonne de gel de silice en éluant avec un mélange chlorure de méthylène/méthanol 98/2. On obtient 800 mg du composé indiqué ci-dessus. P.f. 97-98°C.

### b) Dichlorhydrate de 2-(3-aminopyrrolidin-1-yl)-6-chloropyrimidine.

On dissout le composé obtenu à l'étape a) ci-dessus dans 10 ml d'éthanol saturé de chlorure d'hydrogène et on agite à température ambiante pendant une nuit. On filtre le précipité qu'on cristallise ensuite dans l'éthanol absolu pour obtenir 0,56 g du composé indiqué dans le titre. P.f. > 330°C.

### EXEMPLE 27

*Chlorhydrate de 2-(3-aminopyrrolidin-1-yl)-6-chloropyridine*

### a) 2-(3-acétylaminopyrrolidin-1-yl)-6-chloropyridine.

On chauffe à reflux pendant 5 heures un mélange de 4,8 g (0,0375 mol) du composé obtenu dans la PREPARATION VI, 5,54 g (0,0371 mol) de 2,6-dichloropyridine et 6,5 g (0,047 mol) de carbonate de potassium anhydre dans 90 ml d'alcool n-amylique. On refroidit, on filtre et on concentre le filtrat à sec. On triture le résidu dans de l'eau (50 ml), on filtre, on lave le résidu sur filtre à l'eau, on le reprend dans de l'éther isopropylique, on évapore à sec et on cristallise dans 60 ml d'acétate d'éthyle, pour obtenir 5,7 g du composé indiqué ci-dessus.

### b) Chlorhydrate de 2-(3-aminopyrrolidin-1-yl)-6-chloropyridine.

On chauffe à reflux pendant 24 heures un mélange de 4,46 g (0,187 mol) du composé obtenu dans l'étape a) ci-dessus et 17,8 g (0,30 mol) d'hydroxyde de potassium en poudre dans 18,3 ml d'eau et 90 ml d'éthanol à 95%. On évapore jusqu'à un petit volume, on extrait à l'éther éthylique, on évapore l'éther et on dissout le résidu dans un mélange de 15 ml d'alcool isopropylique et 10 ml d'éther éthylique. On y ajoute de l'isopropanol saturé de chlorure d'hydrogène et on récupère le chlorhydrate (2,3 g) par filtration. P.f. 258-260°C.

### EXEMPLES 28 - 33

En suivant essentiellement la procédure de l'Exemple 25, mais en remplaçant la 2,6-dichloropyrazine par la 2,6-dibromopyridine, la 2,3-dichloropyridine, la 2,5-di-chloropyridine, la 2-chloro-6-trifluorométhylpyridine, la 2-chloro-6-méthoxypyridine et la 2-chloro-6-méthylpyridine on obtient, respectivement
la 2-(3-aminopyrrolidin-1-yl)-6-bromopyridine (Ex. 28),
la 2-(3-aminopyrrolidin-1-yl)-3-chloropyridine (Ex. 29),
la 2-(3-aminopyrrolidin-1-yl)-5-chloropyridine (Ex. 30),
la 2-(3-aminopyrrolidin-1-yl)-6-trifluorométhylpyridine (Ex. 31),
la 2-(3-aminopyrrolidin- 1 -yl)-6-méthoxypyridine (Ex. 32),
la 2-(3-aminopyrrolidin-1-yl)-6-méthylpyridine (Ex. 33),
sous forme de sels d'addition à l'acide chlorhydrique.

### EXEMPLE 34

*Chlorhydrate de 2-(3-amino-3-méthylpyrrolidin-1-yl)-6-chloropyridine*

En suivant la procédure de l'Exemple 27, mais en utilisant la 3-acétylamino-3- méthylpyrrolidine décrite dans EP-132845 ("Reference Example 7") au lieu du composé de la PREPARATION VI, à l'étape a), on obtient le chlorhydrate de 2-(3-amino-3-méthylpyrrolidin-1-yl)-6-chloropyridine.

### EXEMPLE 35

*Oxalate de 2-(3-diméthylaminopyrrolidin-1-yl)-6-chloropyridine*

On dissout 0,25 g (0,00127 mol) du composé de l'Exemple 27 sous forme de base libre dans 0,25 ml d'acide formique à 85% (0,00564 mol). On y ajoute 0,3 ml d'aldéhyde formique à 38% (0,04 mol) et on chauffe à 100°C pendant 2 heures. On rend basique à l'aide d'une solution de NaOH et on extrait à l'acétate d'éthyle. On sèche les extraits organiques et on évapore le solvant sous vide. On dissout le résidu dans une petite quantité d'isopropanol et on y ajoute à chaud une solution d'acide oxalique dans l'isopropanol. On laisse refroidir et on recupère le produit indiqué dans le titre par filtration.

### EXEMPLE 36

*Chlorhydrate de 2-(3-aminopyrrolidin-1-yl)-6-vinylpyridine*

### a) 2-(3-tertbutoxycarbonylaminopyrrolidin-1-yl)-6-vinylpyridine.

On obtient le composé indiqué ci-dessus en suivant essentiellement la procédure décrite à l'étape a) de l'Exemple 19 mais à partir du 2-(3-tertbutoxycarbonylaminopyrrolidin-1-yl)-6-bromopyridine, obtenu par réaction de la 2,6-dibromopyridine avec le composé de la PRÉPARATION VII, au lieu de la 2-(3-tertbutoxy carbonylaminométhylazétidin-1-yl)-6-bromopyridine.

### b) Chlorhydrate de 2-(3-aminopyrrolidin-1-yl)-6-vinylpyridine

A partir du composé obtenu à l'étape a) ci-dessus et en suivant la procédure de l'Exemple 22, on obtient le composé indiqué dans le titre.

### EXEMPLE 37

*Chlorhydrate de l'ester éthylique de l'acide* *6-(3-aminopyrrolidin-1-yl)-2-pyridinecarboxylique*

On obtient le composé indiqué ci-dessus en suivant la procédure de l'Exemple 20 mais en remplaçant le composé de la PRÉPARATION II par la 3-tertbutoxycarbonylamino pyrrolidine.

### EXEMPLE 38

*Oxalate de 6-(3-aminopyrrolidin-1-yl)-2-pyridinecarbonitrile*

On obtient le composé indiqué dans le titre en suivant essentiellement la procédure de l'Exemple 20 mais en remplaçant le dichlorhydrate de la 3-aminométhylazétidine par le dichlorhydrate de la 3-aminopyrrolidine obtenue à partir du composé de la PRÉPARATION VI par hydrolyse.

### EXEMPLE 39

*Chlorhydrate de 2-[(3-aminométhyl)pyrrolidin-1-yl]-6-chloropyridine*

On chauffe à la température de reflux pendant 6 heures un mélange de 1,5 g (0,0075 mol) du composé de la PRÉPARATION V, 1,12 g (0,0075 mol) de 2,6-dichloropyridine, et 2,5 g (0,0184 mol) de K₂CO₃ dans 15 ml d'alcool n-amylique. On refroidit, on filtre les sels et on évapore le solvant sous vide. On obtient un produit huileux qui est purifié par chromatographie sur colonne en éluant avec un mélange CH₂Cl₂/acétate d'éthyle 8/2. On dissout le résidu dans de l'éthanol saturé d'HCl et après une nuit sous agitation à température ambiante on filtre le précipitat et on le cristallise d'un mélange EtOH/H₂O 30/1. P.f. 219°C.

## Revendications

1. 1-Hétéroaryl-azétidine ou -pyrrolidine de formule (I) 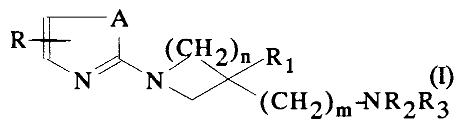 dans laquelle
A représente un groupement -CH=CH-, -CH=N- ou -N=CH- ;
R représente un atome d'hydrogène, un atome d'halogène, un groupe (C₁-C₄)alkyle, (C₁-C₄)alcoxy, (C₁-C₄)alkylthio, un groupe cyano, carboxamido, trifluorométhyle, vinyle, formyle, un groupe carboxy libre, salifié ou estérifié, un groupe hydroxy, hydroxyméthyle, mercapto, un groupe amino, mono- ou di-(C₁-C₄)alkylamino,aminométhyle, mono- ou di-(C₁-C₄)alkylaminométhyle, 1-pipéridino, 1-pyrrolidino, 1-pipérazino ou 4-(C₁- C₄)alkyl-1-pipérazino, ce groupe R pouvant remplacer un quelconque des atomes d'hydrogène du noyau hétéroarylique;
R₁ est un atome d'hydrogène ou un groupe méthyle;
R₂ et R₃, identiques ou différents, représentent un atome d'hydrogène ou un groupe (C₁-C₄)alkyle;
n est 1 ou 2, m est 0 ou 1; et m + n ≧ 2
ainsi que ses sels d'addition avec des acides minéraux ou organiques.

2. 1-Hétéroaryl-azétidine selon la revendication 1 où n = m = 1 de formule (Ia) 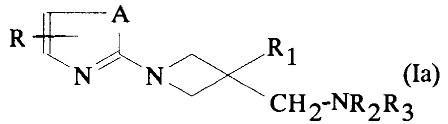 dans laquelle A, R, R₁,R₂ et R₃ sont tels que définis dans la revendication 1, ainsi que ses sels d'addition d'acides.

3. 1-Hétéroaryl-pyrrolidine selon la revendication 1 où n = 2 de formule (Ib) 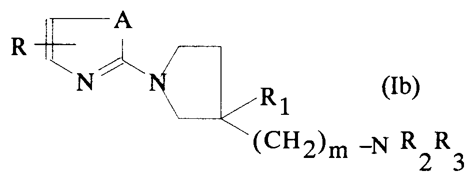 dans laquelle m, A, R, R_{1,} R₂ et R₃ sont tels que définis dans la revendication 1, ainsi que ses sels d'addition d'acides.

4. 1-Hétéroaryl-azétidine de la revendication 2 où A représente un groupement -CH=CH-, ou -N=CH-, R, R₂ et R₃ sont tels que définis dans la revendication 2 et R₁ est l'hydrogène, ainsi que ses sels d'addition d'acides.

5. 1-Hétéroaryl-azétidine de la revendication 4 où R représente un atome d'hydrogène ou d'halogène, un groupe (C₁-C₄)alkyle, (C₁-C₄)alcoxy, cyano, carboxamido, trifluorométhyle, vinyle, formyle, carboxy libre, salifié ou estérifié, ou amino et A, R₁, R₂ et R₃ sont tels que définis dans la revendication 4, ainsi que ses sels d'addition d'acides.

6. 1-Hétéroarylazétidine de la revendication 5 où A représente un groupement -CH=CH-, R représente un atome de chlore ou de brome aux positions 3-, 5- ou 6-, R₁ est un atome d'hydrogène et R₂ et R₃ sont identiques et représentent un atome d'hydrogène ou un groupe (C₁-C₄)alkyle, ainsi que ses sels d'addition d'acides.

7. 1-Hétéroaryl-pyrrolidine de la revendication 3 où A représente un groupement -CH=CH- ou -N=CH-, R₁ est un atome d'hydrogène et m, R, R₂ et R₃ sont tels que définis dans la revendication 3, ainsi que ses sels d'addition d'acides.

8. 1-Hétéroaryl-pyrrolidine de la revendication 7 où R représente un atome d'hydrogène,d'halogène, un groupe (C₁-C₄)alkyle, (C₁-C₄)alcoxy, cyano, carboxamido, trifluorométhyle, vinyle, formyle, carboxy libre, salifié ou estérifié, ou amino et A, m, R₁, R₂ et R₃ sont tels que définis dans la revendication 7 ainsi que ses sels d'addition d'acides.

9. 1-Hétéroaryl-pyrrolidine de la revendication 8 où A représente un groupement -CH=CH-, R représente un atome de chlore ou de brome aux positions 3-, 5- ou 6-, m est 0, R₁ est un atome d'hydrogène et R₂ et R₃ sont identiques et représentent un atome d'hydrogène ou un groupe (C₁-C₄)alkyle, ainsi que ses sels d'addition d'acides.

10. Composition pharmaceutique renfermant à titre de principe actif au moins un composé de formule (I) 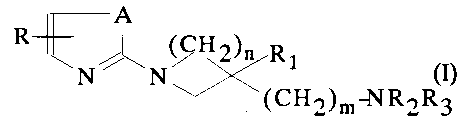 dans laquelle A, R, R₁, R₂, R_{3,} n et m sont tels que définis dans l'une quelconque des revendications 1 à 9 ou un de ses sels d'addition avec des acides pharmaceutiquement acceptables en association avec un véhicule pharmaceutiquement acceptable.

11. Utilisation d'un composé de formule (I) 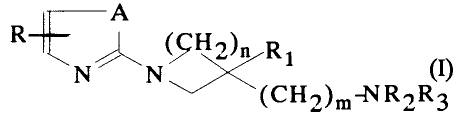 dans laquelle A, R, R₁, R₂, R₃, m et n sont tels que définis dans l'une quelconque des revendications 1 à 9 ou d'un de ses sels d'addition avec des acides pharmaceutiquement acceptables pour la préparation d'un médicament destiné à la prophylaxie et au traitement des troubles qui impliquent le système sérotoninergique soit périphérique soit central, lorsque l'on souhaite avoir une action agoniste sélective médiée par les récepteurs 5-HT₃ à la sérotonine.

12. Utilisation selon la revendication 11 pour l'obtention d'un médicament destiné à la prophylaxie et au traitement des troubles dysthimiques, des troubles psychotiques, des cas d'anxiété ou de la constipation.

13. Procédé pour la préparation d'un composé de formule (I) 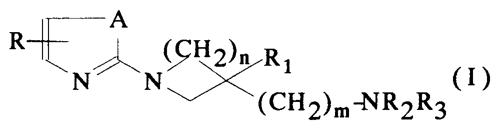 dans laquelle
A représente un groupement -CH=CH-, -CH=N- ou -N=CH-;
R représente un atome d'hydrogène, un atome d'halogène, un groupe (C₁-C₄)alkyle, (C₁-C₄)alcoxy, (C₁-C₄)alkylthio, un groupe cyano, carboxamido, trifluorométhyle, vinyle, formyle, un groupe carboxy libre. salifié ou estérifié, un groupe hydroxy, hydroxyméthyle, mercapto, un groupe amino, mono- ou di-(C₁-C₄)alkylamino,aminométhyle, mono- ou di-(C₁₋ C₄)alkylaminométhyle, 1-pipéridino, 1-pyrrolidino, 1-pipérazino ou 4-(C₁-C₄)alkyl-1-pipérazino, ce groupe R pouvant remplacer un des quelconques atomes d'hydrogène du noyau hétéroarylique;
R₁ est un atome d'hydrogène ou un groupe méthyle;
R₂ et R₃, identiques ou différents, représentent un atome d'hydrogène ou un groupe (C₁-C₄)alkyle;
n est 1 ou 2, m est 0 ou 1 et m + n ≧ 2;
et ses sels d'addition avec des acides minéraux ou organiques;
caractérisé en ce que
- on fait réagir un composé hétérocyclique halo-substitué de formule (II) 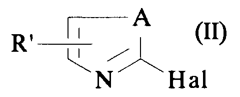 dans laquelle A a la signification donnée ci-dessus, R' correspond à R ou à un groupe R protégé par un groupe protecteur approprié, facilement éliminable et Hal représente un atome d'halogène,
avec une amine bloquée de formule (III) 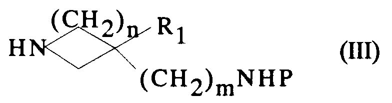 dans laquelle n, m et R₁ sont tels que définis ci-dessus et P est un groupe protecteur temporaire du groupe amino convenablement choisi, pour obtenir un composé intermédiaire de formule (IV) 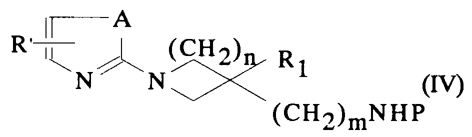 dans laquelle n, m, A, R', R₁ et P sont tels que définis ci-dessus,
- on élimine ensuite les groupes protecteurs présents pour obtenir ainsi un composé de formule(I) dans laquelle A, R et R₁ sont tels que définis ci-dessus et R₂ et R₃ sont l'hydrogène et,
- quand on veut obtenir un composé de formule (I) dans laquelle R₂ et/ou R₃ sont différents de l'hydrogène, on alkyle l'atome d'azote selon des méthodes convenables et,
- éventuellement on convertit le produit final en un de ses sels d'addition avec des acides minéraux ou organiques ; et
- éventuellement après chacune des étapes ci-dessus, on peut convertir un substituant R en un autre selon des réactions connues en soi.

14. 1-Hétéroaryl-azétidine ou -pyrrolidine intermédiaire de formule (IV) 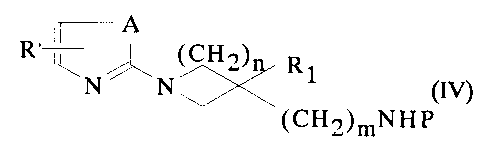 dans laquelle
A représente un groupement -CH=CH-, -CH=N- ou N=CH-;
R' représente un atome d'hydrogène, un atome d'halogène, un groupe (C₁-C₄)alkyle, (C₁-C₄)alcoxy, (C₁-C₄)alkylthio, un groupe cyano, carboxamido, trifluorométhyle, vinyle, formyle, un groupe carboxy libre, salifié ou estérifié, un groupe hydroxy, hydroxyméthyle, mercapto, un groupe amino, mono- ou di-(C₁-C₄)alkylamino,aminométhyle, mono- ou di-(C₁₋ C₄)alkylaminométhyle, 1-pipéridino, 1-pyrrolidino, 1-pipérazino, ou 4- (C₁-C₄)alkyl-1-pipérazino, les groupes hydroxy, mercapto, amino et carboxy pouvant être protégés par des groupes protecteurs facilement éliminables,
R₁ est un atome d'hydrogène ou un groupe méthyle;
n est 1 ou 2, m est 0 ou 1 et m + n ≧ 2, et
P est un groupe protecteur temporaire du groupe amino.

15. 1-Hétéroarylazétidine intermédiaire de la revendication 14 où n est 1, m est 1 et A, R', R₁ et P sont tels que définis dans la revendication 14.

16. 1-Hétéroaryl-pyrrolidine intermédiaire de la revendication 14 où n est 2 et m, A, R', R₁ et P sont tels que définis dans la revendication 14.
